# EUROPEAN PATENT SPECIFICATION

(11) **EP 1 646 721 B1**
(45) Date of publication and mention of the grant of the patent: **16.11.2011**
(21) Application number: 04736419.5
(22) Date of filing: 09.06.2004
(51) Int. Cl.: C12N 15/82, C12N 9/06

(54) **A METHOD OF SELECTIVELY PRODUCING MALE OR FEMALE STERILE PLANTS**
VERFAHREN ZUR SELEKTIVEN HERSTELLUNG MÄNNLICH- ODER WEIBLICH-STERILER PFLANZEN
PROCEDE DE PRODUCTION SELECTIVE DE PLANTES STERILES MALES OU FEMELLES

(30) Priority: 08.07.2003 GB 0316040; 28.01.2004 GB 0401839
(43) Date of publication of application: 19.04.2006
(73) Proprietor: Syngenta Limited, Guildford Surrey GU2 7YH (GB)
(72) Inventor: DALE, Richard, Bracknell, Berks RG42 6EY (GB); HAWKES, Timothy Robert, Bracknell, Berkshire RG42 6ET (GB); THOMPSON, Paul Anthony, Bracknell, Berkshire RG42 6ET (GB); VINER, Russell, Bracknell, Berkshire RG42 6ET (GB)
(74) Representative: Palmer, Sarah Louise
(86) International application number: PCT/GB2004/002447
(87) International publication number: WO 2005/005641

(56) References cited:
- WO-A-01/29237
- WO-A-98/39462
- WO-A-02/072804
- WO-A-03/060133
- WO-A-03/072792
- PILONE M S: "D-amino acid oxidase: new findings" CMLS CELLULAR AND MOLECULAR LIFE SCIENCES, BIRKHAUSER VERLAG, BASEL, CH, vol. 57, 2000, pages 1732-1747, XP002967442 ISSN: 1420-682X
- SACCHI SILVIA ET AL: "Engineering the substrate specificity of D-amino-acid oxidase" JOURNAL OF BIOLOGICAL CHEMISTRY, vol. 277, no. 30, 28 July 2002 (2002-07-28), pages 27510-27516, XP002295815 ISSN: 0021-9258
- ERIKSON OSKAR ET AL: "A conditional marker gene allowing both positive and negative selection in plants" NATURE BIOTECHNOLOGY, NATURE PUBLISHING, US, vol. 22, no. 4, April 2004 (2004-04), pages 455-458, XP002292188 ISSN: 1087-0156

### SEQUENCE LISTING

<110> Syngenta Limited
<120> A method of selectively producing male or female sterile plants
<130> PPD70629
<160> 7
<170> Patent In version 3.1
<210> 1
   <211> 27
   <212> DNA
<213> Artificial Sequence
<220>
   <223> Primer
<400> 1
   aactgcagct ttttggttag cgaatgc 27
<210> 2
   <211> 35
   <212> DNA
   <213> Artificial Sequence
<220>
   <223> Primer
<400> 2
   cagactagtt ttagctaatt tctttaagta aaaac 35
<210> 3
   <211> 1107
   <212> DNA
   <213> Rhodotorula gracilis
<400> 3
   atgggatccc aaaagagggt tgtggtgctg ggttccggcg tgataggact cagctccgcg 60
   cttatacttg cccggaaggg gtactccgtc cacatcctgg cccgggacct cccagaggat 120
   gttagctcac agaccttcgc gtccccttgg gctggagcca actggacccc ttttatgacc 180
   ctcactgacg gcccgaggca ggcaaagtgg gaggagtcta cattcaagaa gtgggtggaa 240
   cttgtgccaa cggggcatgc catgtggttg aagggaacca ggcgtttcgc ccaaaatgag 300
   gacggactgc tcggtcactg gtacaaagat atcaccccca attatagacc cttgccctct 360
   tccgaatgtc caccaggcgc tattggcgtg acttatgaca cattgtcagt gcacgctcca 420
   aagtactgcc aatacctcgc aagggagctc cagaagctgg gggcgacatt cgagcgccgc 480
   accgttactt ccctcgagca agcttttgat ggggctgacc tcgtcgttaa cgcgacgggg 540
   ctgggtgcca agtccatcgc tggcatcgat gaccaggcgg ccgagcctat tcgcggtcaa 600
   acggtgctcg tcaagtcgcc ctgcaaaagg tgtactatgg acagctcgga cccggcatca 660
   ccggcgtaca tcatcccgcg gccaggaggc gaagtgattt gcggcggtac gtacggggtc 720
   ggagactggg atctctcggt caacccagag accgtccagc gcatcctcaa acactgcctg 780
   cgcctggatc cgactatttc ttcggacggc acaatcgaag gcatcgaggt gctgcggcat 840
   aacgtcggac tcagaccggc gaggagggga ggccctcgcg ttgaagccga gaggattgtt 900
   cttccacttg acagaacgaa gagccccctc tcactgggcc gtgggagcgc tcgtgcggcc 960
   aaggagaagg aggtgacttt ggtgcatgcc tacggtttct ccagcgctgg ctatcaacag 1020
   tcttggggcg cagccgaaga cgtcgcacaa ttggtcgatg aggcgtttca gaggtatcat 1080
   ggggccgccc gcgagtctaa gctctga 1107
<210> 4
   <211> 120
   <212> DNA
   <213> Artificial Sequence
<220>
   <223> Primer
<220>
   <221> misc_feature
   <222> (22)..(22)
   <223> Where n=a,t, c or g.
<220>
   <221> misc_feature
   <222> (23)..(23)
   <223> Where n=a,t, c or g.
<220>
   <221> misc_feature
   <222> (24)..(24)
   <223> Where n=a,t, c or g.
<220>
   <221> misc_feature
   <222> (97)..(97)
   <223> Where n=a,t, c or g.
<220>
   <221> misc_feature
   <222> (98)..(98)
   <223> Where n=a,t, c or g.
<220>
   <221> misc_feature
   <222> (99)..(99)
   <223> Where n=a,t, c or g.
<400> 4
   tccccatgca agcgatgcac gnnngactcg tccgaccccg cttctcccgc ctacatcatt     60
   ccccgaccag gtggcgaagt catctgcggc gggacgnnng gcgtgggaga ctgggacttg    120
<210> 5
   <211> 120
   <212> DNA
   <213> Artificial Sequence
<220>
   <223> Primer
<220>
   <221> misc_feature
   <222> (22)..(22)
   <223> Where n=a,t, c or g.
<220>
   <221> misc_feature
   <222> (23)..(23)
   <223> Where n=a,t, c or g.
<220>
   <221> misc_feature
   <222> (24)..(24)
   <223> Where n=a,t, c or g.
<220>
   <221> misc_feature
   <222> (97)..(97)
   <223> Where n=a,t, c or g.
<220>
   <221> misc_feature
   <222> (98)..(98)
   <223> Where n=a,t, c or g.
<220>
   <221> misc_feature
   <222> (99)..(99)
   <223> Where n=a,t, c or g.
<400> 5
   caagtcccag tctcccacgc cnnncgtccc gccgcagatg acttcgccac ctggtcgggg     60
   aatgatgtag gcgggagaag cggggtcgga cgagtcnnnc gtgcatcgct tgcatgggga    120
<210> 6
   <211> 7
   <212> PRT
   <213> Artificial Sequence
<220>
   <223> Motif
<400> 6
   Arg Cys Thr Met Asp Ser Ser
   1               5
<210> 7
   <211> 7
   <212> PRT
   <213> Artificial Sequence
<220>
   <223> Motif
<400> 7
   Gly Gly Thr Tyr Gly Val Gly
   1               5

## Description

Heterosis in crop plants can have a marked effect on yield improvement. In general, hybrids exhibit increased yields in comparison with non-hybrid varieties. Hybrids usually give a greater return unit for growth factors such as water and fertilizer. Hybrids often offer superior stress tolerance, uniformity in product and maturity and also afford a simple breeding opportunity to combine characteristics or traits that may be difficult to combine in other ways. Hybrid vigour in plants is generally of sufficient magnitude to warrant commercial exploitation. Commercial hybrids are used extensively in many crops including corn, sorghum, sugar beet, sunflower and canola. However, owing mainly to the lack of economical hybrid seed production methods, wheat, barley and rice are still grown mainly as inbreds.

Traditionally, hybrid seed production involves planting out separate blocks of female and male parent lines with only the seed from the female parents being harvested. To ensure that this seed is hybrid, self pollination of the female parent line must be minimised by rendering the line male-sterile. Methods for making the female parent line male sterile include mechanical, chemical and genetic methods. In diecious plants, such as maize, male sterility can be readily achieved mechanically by detasselling of the male infloresence. However most crops are monoecious and having male and female organs within the same flower makes such physical emasculation impractical. Genetic approaches have therefore sometimes been used.

Genetic male sterility traits which occur are normally controlled by nuclear genes in which the alleles associated with the sterile phenotype are generally expressed recessively with respect to the corresponding alleles associated with fertility. Where genetic male sterility occurs it is normally associated with a single recessive gene that must be homozygous in order for male sterility to be expressed. In order to make practical use of such genetic male sterility traits, breeders usually develop a phenotypically uniform female line that segregates into male-sterile and male-fertile plants. The male fertile plants, once identified, need to be rogued out which is labour intensive. There is always a problem with maintaining the parental line since male fertile plants cannot be eliminated from the population because they are essential for maintenance of the population. Rather than rely on the existence of natural male sterility alleles it is also possible to use molecular biological methods. Plants may be engineered which express, for example, anti-sense or ribozyme genes that decrease or eliminate expression of key genes necessary for the formation of viable pollen. Such transgenic lines of plants are male-sterile and are used for the production of hybrid seed by crossing using pollen from male-fertile plants. The main problem with such lines is that they can only be maintained in a heterozygous state in subsequent generations, via crosses with the isogenic fertile lines. This can be a problem in hybrid seed production where yield is critical. Although, for example by linking herbicide resistance to male sterility, it may be possible to selectively rogue out the male-fertile plants this still necessitates that the plants are planted initially at extra high densities.

The use of cytoplasmic male sterility for commercial hybrid production requires a stable male-sterile cytoplasm and a source of pollen. The cytoplasmic-genetic system of male sterility requires the existence of three types of line for hybrid production, the A line (cytoplasmic male-sterile), B line (male-fertile maintainer) and R line (male fertile with restorer genes). Three-way crosses produced with this system involve maintenance and production of four lines, an A and a B line of one inbred and male-fertile inbreds of the other two. Reliance on a single source of male-sterile cytoplasm can minimise breeding flexibility and lead to progeny with wholesale susceptibility to particular diseases.

Hybrid seed can also be produced through the use of chemicals that inhibit viable pollen formation. These chemicals, called gametocides, are used to impart transitory male- sterility. However the expense, registerability and reliability of gametocides has limited their use.

A shortcoming of traditional hybrid seed production systems is the need to plant separate rows or blocks of the male and female parent lines. Here low efficiency pollination is an especially acute problem in crop species, such as wheat, that release small amounts of pollen which does not travel far on the wind. In such crops as much as two/thirds of the hybrid-producing field needs to be dedicated to male pollen-donor plants and then hybrid seed production therefore becomes uneconomic.

In order to achieve more economic seed production in wheat and other crops it is necessary to move male and female plants closer together for more efficient pollen transfer; most efficiently by interplanting males and females within centimetres of each other in the same rows. In such a system it would be impractical to harvest only the seed from the (male-sterile) female parents. The contamination with non-hybrid seed originating from the male parent can be minimised by using as low a percentage of such male parent plants in the planting mix as possible and/or by using male plants which are female sterile. A method for constructing a dominant female sterile line has been described (EP 412,006 A1 (1990); Goldman et al., (1994) EMBO. J., 13, 2976-2984) but, as with the male sterile lines, the line has to be maintained as a heterozygote.

Accordingly there remains a need for simple economic methods of hybrid seed production. In particular, in order efficiently to produce hybrid seed there remains a need to provide both male-sterile female parental lines and female-sterile male parental lines which can be easily maintained as pure homozygous lines and which are useful for efficient hybrid seed production. Methods which are described in the art for achieving this include methods wherein hybrid seed is produced from male and female parent lines at least one of which comprises a heterologous chimeric gene, preferentially expressed in floral tissue, which renders the line conditionally sterile dependent upon the exogenous application of a non-phytotoxic substance which can be specifically and locally converted to a phytotoxin by an enzyme which is encoded by the heterologous chimeric gene and which is preferentially expressed in either the male or female reproductive structures. The non-phytotoxic substance may be a pro-herbicide. The advantage of having such conditionally sterile parent lines is that it allows them to be maintained as homozygotes with respect to the sterility trait. Fertility is only disrupted upon exogenous application of the non-phytotoxic substance. In one such example of a conditional male sterility system a gene encoding a deacetylase enzyme is preferentially expressed in tapetal cells of male flower tissue where it converts the exogenously applied pro-herbicide N-acetyl L phosphinothricin to the phytotoxin L phosphinothricin and thus prevents viable pollen formation. In further similar examples: (i) tapetum preferential expression of a bacterial cytochrome P450 catalyses conversion of pro-herbicide R7402 to a sulphonylurea phytoxin which prevents the production of viable pollen; and (ii) tapetum preferential expression of a phosphonate monoester hydrolase catalyses conversion of glyceryl glyphosate pro-herbicide to the phytotoxin glyphosate which also prevents production of viable pollen. WO 98/03838 describes examples of a conditional female sterility system wherein enzymes capable of converting the pro-herbicides to phytoxins are preferentially expressed in female reproductive structures.

Despite the existence of these methods for making male and female parent lines that are conditionally sterile, hybrid seed production remains far from routine in crops such as wheat. The current inventions concern*, inter alia,* improvements in the art with respect to the generation of female parent lines which are conditionally male sterile and male parent lines which are conditionally female sterile.

The current invention relates to improvements in methods for the production of crop hybrid seed. In particular the invention relates to a method of hybrid seed production from male and female parent lines at least one of which is conditionally female or male sterile dependent upon the exogenous application of a substance which is non-phytotoxic to the crop and which include pro-herbicides. The invention further relates to a method in which the said non-phytotoxic substance is applied at a time and in sufficient amount that self fertilization is minimised or prevented in the conditionally sterile parent line(s). The current invention also relates to a method of generating conditionally male or female-sterile plants by i) transforming plant material with one or more chimeric genes which, singly or together, encode one or more enzymes capable of reacting with a non-phytotoxic substance, preferably in the form of a pro-herbicide, to produce a phytotoxic one. Enzymes are expressed under operable control of one or more promoters which, in the case of conditionally male sterile plants, causes the enzyme(s) to be expressed preferentially in the male reproductive structures or which, in the case of conditionally female sterile plants, causes the said enzyme(s) to be expressed preferentially in the female reproductive structures. The plant material is regenerated into morphologically normal fertile plants which are conditionally male or female sterile. The invention also includes the use of conditionally male-sterile plants in combination with conditionally female-sterile plants to produce more efficiently hybrid seed, the use, as non-phytotoxic substances, of certain pro-herbicides and the use of chimeric genes to produce more efficiently hybrid seeds, chimeric genes and enzymes useful for the invention. The invention also provides conditionally male-sterile, conditionally female-sterile plants, seeds of these plants and hybrid seeds produced by the method. In preferred embodiments of the invention the crop plants to which the method for making hybrid seed is applied are maize, rice, sorghum, wheat, millet, oats, canola and barley.

According to the present invention there is provided a method of producing male or female sterile plants comprising the steps of transforming plant material with a polynucleotide which encodes at least one enzyme which reacts with a non-phytotoxic substance to produce a phytotoxic one, and regenerating the thus transformed material into a plant, wherein the said non-phytotoxic substance is applied to the plant up to the time of male or female gamete formation and/ or maturation, so that the non-phytotoxic substance provides for the production of a phytotoxic one which selectively prevents the formation of or otherwise renders the said gametes non-functional, wherein the enzyme is expressed preferentially in either male or female reproductive structures, characterised in that (i) the non-phytotoxic substance is selected from the group consisting ofD-alpha amino acids, peptide derivatives of non-protein D-alpha amino acids and (ii) the enzyme is a mutant form of a D-amino acid oxidase which has a lysine at the position corresponding to the phenylalanine at position 58 of wild-type Rhodotorula gracilis D-amino acid oxidase.

Since it is a desirable objective to maximise the yield of hybrid seed and therefore to minimise any crop damage, in preferred embodiments, the non-phytotoxic substance is a proherbicide selected from amongst compounds which are relatively non-phytotoxic to the crop. In order to be capable of an effect against floral tissues it is also desirable that pro-herbicides be progenitors of phyto-toxins that are effective in 'non-green' tissues. Thus, in preferred embodiments of the invention, pro-herbicides are selected from those which are progenitors of phyto-toxins which are directly phytotoxic to non-green tissues rather than those which have a principle site of action in photosynthesis or in the generation of photosynthetic pigments. It is also a desirable objective to minimise the costs of hybrid seed production. Thus, in preferred embodiments, pro-herbicides are selected from amongst those chemical substances for which approval from appropriate regulatory authorities for use in crops is either already granted or is pending.

### Nomenclature: Definitions

'Gene' as used herein refers to any DNA sequence comprising several operably linked DNA fragments such as a promoter and a 5' regulatory region, a coding sequence and an untranslated 3' region comprising a polyadenylation site.

'Chimeric' when referring to a gene or DNA sequence is used to refer to the fact that in nature, the coding sequence is not associated with the promoter or with at least one other regulatory region of the DNA in the gene.

'Chimeric gene' as used herein refers to a gene wherein, in nature, the coding sequence is not associated with the promoter or with at least one other regulatory region of the DNA in the gene.

'Expression cassette' as used herein refers to a transferable region of DNA comprising a chimeric gene which is flanked by one or more restriction or other sites which facilitate precise excision from one DNA locus and insertion into another.

'Non-phytotoxic substances' are, in the context of the current invention, substances which are relatively non-phytotoxic to plants, cells or tissues of any particular crop to which the method of the invention is applied. Non-phytotoxic substances need not be non-phytoxic in all plant tissues of all plants. Non-phytotoxic substances include pro-herbicides which are substances with no appreciable *direct* toxic effect on plant tissues but which are progenitors of active phyto-toxins. In susceptible plant species such pro-herbicides act indirectly as herbicides through the action of endogenous enzymes which convert them *in planta* to a phyto-toxin.

'Phyto-toxins' are, in the context of the current invention, substances which are toxic to plants, plant tissues and plant cells of the particular crop to which the method of the invention is applied. Such phyto-toxins need not be phyto-toxic to all plant tissues from all plant species.

'Female reproductive structure' means the female gametes and those portions of the plant that are specialised for the production, maturation and viability of female gametes. Normally this comprises those portions of a plant that comprise the carpel or gynoecium ("pistill"). The carpel of a plant includes but is not limited to, a stigma, style, ovary and cells or tissues that are comprised by the stigma, style and ovary.

'Male reproductive structure' means the male gametes and those portions of the plan that are specialised for the production, maturation and viability of male gametes. This comprises those portions of a plant that comprise, for example, microspores, stamens, tapetum, anthers and the pollen.

'Female-sterile plant' as used herein is a plant that is incapable of supporting viable seed formation when pollinated with functional or viable pollen. Such female sterility can be the result of breeding selection or the presence of a transgene. A 'conditionally female-sterile plant' refers to a plant which under normal growing conditions is female fertile and which can become female-sterile under specific conditions. In the context of the current invention the said conditions comprise the exogenous application of a pro-herbicide or other non-phytotoxic substance. In the context of the current invention such a 'female-sterile plant' or 'conditionally female-sterile plant' remains male fertile and able to produce viable pollen.

'Male-sterile plant' as used herein is a plant that is incapable of supporting viable pollen formation. Such male sterility can be the result of breeding selection or the presence of a transgene. A 'conditionally male-sterile plant' refers to a plant which under normal growing conditions is male fertile and which can become male-sterile under specific conditions. For example the conditions might comprise physical emasculation or application of a specific chemical gametocide. In the context of the current invention the said conditions particularly comprise the exogenous application of a pro-herbicide or other non-phytotoxic substance. In the context of the current invention such a 'male-sterile plant' or 'conditionally male-sterile plant' remains female fertile and able to produce viable seeds when pollinated with functional or viable pollen.

'Promoter region' as used herein is a region of DNA comprising at least a functional promoter and, optionally, some or all of its associated upstream regulatory sequences including enhancer sequences and/or associated downstream sequences including some or all of the 5' untranslated region of the gene endogenous to the promoter.

'Inter-planting' as used herein refers to a method of planting seeds or plants in a field that ensures adequate cross-pollination of male sterile or conditionally male-sterile plants by the male-fertile plants. This can be achieved either by random mixing of female and male parent seed in different blends (80/20; 90/10; etc) before planting or by planting in specific field patterns whereby different seeds are alternated. When separate harvesting from different plants is required planting in alternating blocks or rows is preferred.

In the method according to the invention the said non-phytotoxic substance may be applied in mixture along with at least one further substance which may be selected from the group consisting of amino acids, safeners, gametocides, glutathione-S-transferase inducers, cytochrome P450 inducers, fertilizers, herbicides, nematocides, synergists, insecticides, fungicides, hormones, plant-growth regulators and cytochrome P450 inhibitors. In particular embodiments the said non-phytotoxic substance may be applied in a mixture with the same phytotoxic substance that the non-phytotoxic substance is a progenitor of.

The non-phytotoxic substance is a D-amino acid and, in particular, it may be the D enantiomer of phosphinothricin, the D enantiomer of bialaphos or selected from the group consisting of D-aspartate and D-glutamate.

The D-amino acid oxidases are mutant forms of the enzyme from Rhodotorula gracilis. Such mutants always have a lysine at position 58 (ie they are F58K mutants of the wild-type sequence) and comprise further single, double or triple amino acid substitutions at positions 213, 223 and 238 when compared with the wild type sequence. At position 213 the wild-type methionine is replaced by, Thr, Gly, or, Gln and/or the wild-type tyrosine at position 223 is replaced by Thr, Gly, Gln, Cys or Asn and/or the wild type tyrosine at position 238 is replaced by Thr, Gly, or Gln . In a particularly preferred embodiment the methionine at position 213 is replaced by threonine.

Where the non-phytotoxic substance is a D-amino acid other than D-phoshinothricin or D-bialaphos then the D-amino acid is preferably, not an endogenous plant metabolite and is selected to be one that is phloem mobile, metabolically stable in the plant (preferably having a t ½ in the plant of greater than ~ 1 week) and an efficient substrate of the D-amino acid oxidase. Oxidation of the D-amino acid by the enzyme is concomitant with reduction of oxygen to phytotoxic peroxide anions.

In a preferred embodiment the oxidase enzyme is targeted to a subcellular location other than the peroxisome. This is achieved, for example, by modifying the gene so that three C-terminal amino acids (e.g. SKL in the case of the the Rhodotorula gracilis-derived D-amino acid oxidase) are deleted or modified and/or by addition of sequence to add a chloroplast or mitochondrial transit peptide to the N-terminus.

Further suitable D-amino acid oxidases may be obtained preferably from fungal sources, by the mutation and selective procedures known to the skilled man and augmented by the present disclosure.

Further mutant D-amino acid oxidase enzymes and DNA coding sequences suitable for working the invention are obtained by expressing libraries of candidate mutant D-amino acid oxidases in a suitable host cell such as *E.coli* or a yeast (suitable host strains lack an endogenous oxidase or dehydrogenase activity versus D-phosphinothricin) for transformation to a phenotype with increased sensitivity to growth inhibition by D-phosphinothricin on a minimal medium. This method relies upon the ability of transformed *E.coli* clones to produce L-PPT from D-PPT via the combined action of their endogenous L transaminase activity and the heterologously expressed oxidase. Alternatively, suitable and improved genes are selected on the basis of in vitro assay of the expressed enzyme for the desired ability to oxidise D-phosphinothricin. There are many methods for directly assaying the activities of D-amino acid oxidases such as based upon detection of peroxide (Enzyme Microb. Technol., (2000), 27(8), 605-611), depletion of oxygen using an oxygen electrode or based on direct detection of ammonia or of the keto-acid product.

It is disclosed that, a fungally-derived DAMOX gene is cloned into a shuttle vector under operable control of a promoter (e.g GAL promoter) capable of expression in the host organism in which the selection will be carried out (preferably yeast). This gene is then subjected to mutagenesis, for example by Mn2+-poisoned PCR; plasmid DNA replication in a strain which is defective in DNA repair/ editing processes such as *E.coli* strain XL1 red; or by plasmid DNA replication in a host strain which is subjected to mutagenesis using, for example X-Rays, UV light, addition of a chemical mutagen and transformed into a host organism (preferably yeast). The desired DNA encoding a DAMOX having the desired property of an enhanced ability to oxidise D-PPT is selected for (following an optional, initial selection step for transformants based upon selectable markers present on the shuttle vector allowing, for example, selection *via* restoration of prototrophy or growth in presence of hygromycin etc) *via*, for example
a) Selection of transformed cells having the ability to utilise amino acids which are chemically similar to D-phosphinothricin as sole nitrogen source. For example, transformed yeast colonies are selected which are able to grow on analogues ofD-PPT (and its esters) where the phosphinic acid moiety is replaced with a carboxylate (i.e D-glutamate), sulphonate, phosphonate, sulphone, or sulfoxide moiety (or esters of these) as sole N source. E.g.. 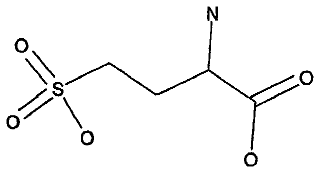 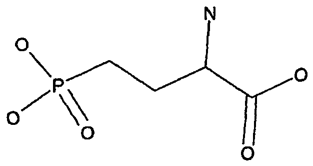 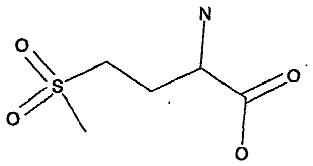
b) Selection of transformed cells capable of utilizing D-PPT itself as sole N source. For this selection the host cell is also transformed with a gene capable of negating the inhibitory effect of L-phosphinothricin on glutamine synthetase For example the shuttle vector also comprises a gene which encodes an enzyme such as PAT which inactivates L-PPT.

Cycles of mutation and selection may be iterated. D-amino acid oxidases may further be cloned, expressed, part purified and characterised kinetically in order to identify genes and DAMOXs with the most suitable properties (e.g enzyme stability, high kcat/ Km value for oxidation of D-PPT, minimal oxidation of any endogenous plant substrates, optimum pH etc).

Where the non-phytoxic substance is D-phosphinothricin (PPT) it may be obtained from a mixture of D and L PPT. For example, DL PPT may be added to a culture medium (preferably minimal) of *E.coli* cells (optionally an arg E mutant to minimise the background level of N-acetyl PPT deacetylase activity) where the *E.coli* is transformed to express a PAT gene (encoding an enzyme which transfers an acetyl group from acetyl CoA to L-PPT) at a high level (e.g inducibly, upon addition of IPTG). Preferably, the *E.coli* is also engineered to express acetyl CoA synthetase. After allowing a suitable time for the L component of the phosphinothricin to substantially all be N-acetylated, (judged, for example, by monitoring the conversion using 31-P NMR) D-PPT is recovered and purified from the cell-free medium using successive steps of, for example, solvent extraction at high and low pH, anion and cation exchange chromatography, selective crystallisation with chiral cations such as chinchocine or other procedures known in the art such as liquid/ liquid extraction with two non-miscible aqueous phases as the phase system (cf methods in USP 5,153,355). Typically a late step is cation exchange chromatography from which D-PPT is recovered as the ammonium salt.

Alternatively, D-PPT may be obtained by an enzymatic method wherein DL PPT + 2-ketoglutarate is converted to primarily a mixture of D-PPT, 2-oxo PPT (and its decarboxylation products) and GABA by the combined actions of (I) L-aminotransferase (e.g from *E.coli*) and (II) glutamate decarboxylase. The desired pure D-PPT is resolved from the reaction mixture using methods known in the art and as outlined above.

D-PPT may also be obtained using an enzymatic method wherein DL PPT + 2-ketoglutarate + NAD is converted to primarily a mixture of D-PPT, 2-oxo PPT (and its decarboxylation products) NADH, and ammonia by the combined actions of (I) L-aminotransferase and (II) glutamate dehydrogenase. The desired D-PPT is purified from the reaction mixture.

In a yet further method of making D-PPT, DL PPT is treated with a L amino acid oxidase so that the only remaining amino acid is the desired D form. This D-PPT is then purified from the reaction mixture.

A still further method involves (I) conversion of DL PPT to N-acetyl DL PPT (using acetic anhydride or other acetylating reagents and methods well known in the art) and (II) treatment of N-acetyl DL PPT with D-aminoacylase so that only N-acetyl-D-PPT is deacetylated. The resultant D-PPT is purified from the reaction mixture. For example, D-PPT is resolved from N-acetyl-L-PPT by binding to Dowex anion exchange resin and elution with 40 mM formic acid. Under suitable loading conditions this acid elutes the D-PPT whilst leaving the N-acetyl L-PPT bound to the column.

A still further method involves treatment ofDL PPT with L-aminoacylase and an acylating agent in a non-aqueous solvent so that only the desired D-PPT is left in a non - acetylated form.

A yet further method of preparing pure D-PPT involves enantioselective crystallisation from DL PPT using a chiral base such as chinchocine and addition of a seed crystal of the chiral base with pure D-PPT.

A yet further method of preparing pure D-PPT from DL-PPT by direct chiral chromatography using a chiral base column.

A detailed method for the production of pure D-PPT is given in one of the Examples following.

DNA sequences encoding the enzymes used in the present invention may, optionally, be further mutated and selected in order to generate further useful enzymes having improved utility. Many characteristics of enzymes are thus improved including catalytic activity (kcat/ Km) versus the desired substrate, temperature stability and pH optimum. Methods for generating, screening and selecting for such improved variants are well known. For example, suitable variant DNA sequences are generated by a process of mutagenesis (e.g by passaging DNA through bacterial or yeast strains with error-prone DNA replication such as *E.coli* XL1 red, by UV, chemical or targeted oligonucleotide PCR mutagenesis). In particular such genes are produced by any of a number of alternative processes of DNA shuffling or 'sexual PCR' as, for example, summarised in WO 00/61740 from pages 28-41. Many methods are suitable for selecting such improved genes. Genes may be suitably expressed in a suitable host cell such as *E.coli* or yeast and selected for improvement using suitable such assays as, for example, described herein.

The chimeric genes encoding enzymes for use in the invention which are capable, singly or in combination with others, of converting a non-phytotoxic substance to a phytotoxic one, may each comprise a DNA sequence which encodes one of said enzymes operably linked to a 5' promoter region which preferentially directs expression to either the male or the female reproductive structures. This specificity of expression ensures that the effect of the expressed enzyme(s) will be exerted only within the locality of the tissues and cells necessary for formation of viable seed or viable pollen and will not be deleterious to the plant beyond its effect on fertility in the presence of a suitable non phytotoxic substance, perhaps a pro-herbicide. In addition to promoter regions chimeric genes according to the current invention also comprise a 3' transcriptional terminator sequence. This is responsible for the termination of transcription and correct mRNA polyadenylation. Many such 3' transcriptional terminator sequences are known in the art and are suitable for use in the chimeric genes of the current invention. In particular embodiments the 3' transcriptional terminator sequence is selected from the CMV 35S terminator, the *tml* terminator, the nopaline synthase (nos) terminator and the pea *rbc*S E0 terminator.

5' Promoter regions suitable for use in certain embodiments of the said chimeric genes include 5' regions of genes which are preferentially expressed in female floral tissues. In certain embodiments the 5' promoter region is selected from the group consisting of the stig 1 promoter of tobacco (Goldman et al., (1994) EMBO J., 13, 2976-2984), a modified S13 promoter (Dzelkalns et al (1993) Plant Cell, 5, 8555), the AGL5 promoter (Savidge et al (1995) Plant Cell, 7, 721-733 and the promoter region 5' of the maize-carpel specific ZAG2 gene (Thiessen et al (1995) Gene, 156, 155-166). Optionally, further suitable promoter regions are obtained from regions upstream of the coding sequences of genomic DNA corresponding to cDNA sequences known in the art to be preferentially expressed in female reproductive structures. In certain embodiments such probe cDNAs are selected from the group consisting of the Arabidopsis Fbp7 and Fbp11 genes (Angenent et al., (1995) Plant Cell, 7, 1569-1582) and the orchid-specific cDNAs O40,O108, 039, O126 and O141 (Nadeau et al., (1996) Plant Cell, 8, 213-239). In particular embodiments 5' promoter regions comprising genomic DNA associated with preferential expression in female reproductive structures is selected from DNA regions comprised within the group consisting of the genomic DNA clone pSH64 having the accession number NRRL B-21920, genomic clone, pCIB10302 hybridising to the cDNA P26-A4 having the accession number NRRL B-21655 and genomic DNA clone X2-1 hybridising to cDNA clone P 19-QA having the accession number NRRL B-21919. In further particular embodiments these promoter region comprise nucleotides 1 to 1390 of SEQ ID No. 11, SEQ ID No. 2 and nucleotides 1 to 1093 of SEQ ID No. 4 in WO 98/39462. In further embodiments, further 5' promoter regions suitable for use in the chimeric genes of the invention are isolated and cloned by methods which are familiar to one skilled in the art. For example, novel transcripts expressed in female reproductive structures are identified by isolating RNA from tissues such as maize silks or wheat pistils followed by differential screening using techniques such as differential display, PCR select cDNA subtraction and subtractive cDNA library construction. cDNA clones that are preferentially expressed in the female tissues and not in other parts of the plant such as the leaves, roots and tassels are isolated. The tissue specificity of expression is, optionally, further confirmed by Northern blotting. The cDNA clones are used as probes for genomic library screening. 5' promoter regions and, optionally, 3' untranslated DNA regions associated with tissue preferential expression are obtained from the genomic DNA clones and used in the construction of chimeric genes for preferential expression in female reproductive structures.

5' Promoter regions suitable for use in certain embodiments of the said chimeric genes include 5' regions of genes which are preferentially expressed in male floral tissues. These include promoter regions for expression in pollen, the tapetum or other structures in the anther. In certain embodiments these 5' promoter regions are selected from the group consisting of the LAT52 promoter (Twell et al., (1989) Dev., 109, 705-713), the tomato A127 promoter (Dotson et al., (1996) Plant J., 10, 383-392), the maize Zmg promoter (Hamilton et. al., (1989) Sex. Plant Reprod. 2, 208-212), the maize CDPK promoter (Guerro et al., (1990) Mol. Gen. Genet., 224, 161-168) and the anther specific ant32 and ant43D promoters disclosed in USP 5477002. In certain further embodiments the 5' promoter region is selected from the group consisting of the tapetum-specific promoter CA55 from maize ("Pca55" described in WO 92/13956), the tapetum-specific promoter E1 from rice (described in USP 5639948), the tapetum-specific promoter T72 from rice (described in USP 5639948), the RA8 anther-specific promoter from rice (EMBL/Genbank accession number AF042275; Jeon et al, (1999) PMB, 39, 35-44; WO 00/26389) the anther-specific Tap1 promoter (Spena et al (1992) Theor Appl Genet 84, 520-527) and the ZmC5 - pollen specific promoter from maize (EMBL/Genbank accession number Y13285; Wakeley et al, (1998) PMB, 37, 187-192). Optionally, further suitable promoter regions are obtained from regions upstream of the coding sequences of genomic, DNA corresponding to cDNA sequences, known in the art to be preferentially expressed in male reproductive structures. In certain embodiments such probe cDNAs are selected from the group consisting of the orchid pollen-tube specific cytochrome P450 gene (Nadeau et al., (1996) Plant Cell, 8,213-239), the Bcp1 gene ofArabidopsis (Xu et al (1995) P.N.A.S., 92, 2106-2110) and the male-flower specific MFS14 gene of maize (Wright et al., (1993) Plant J, 3, 41-49). In further embodiments, further 5' promoter regions suitable for use in the chimeric genes of the invention are isolated and cloned by methods which are familiar to one skilled in the art. For example, novel transcripts expressed in male reproductive structures are identified by isolating RNA from tissues such as tassels, pollen tubes, anther or tapetum followed by differential screening by techniques such as differential display, PCR select cDNA subtraction and subtractive cDNA library construction. cDNA clones that are preferentially expressed in the male tissues and not in other parts of the plant such as the leaves, roots and stigma are isolated. The tissue specificity of expression is, optionally, confirmed by Northern blotting. The cDNA clones are used as probes for genomic library screening. 5' promoter regions and 3' untranslated DNA regions associated with tissue preferential expression are obtained from the genomic DNA clones and used in the construction of chimeric genes for preferential expression in male reproductive structures.

Further promoter regions useful in the chimeric genes of the invention include the regions upstream of the Osmads 13 gene of rice, the OSG gene of rice anther, and the YY2 gene of rice. Generally, promoter regions yielding high, early, sustained and preferential expression in male or female reproductive structures are selected as most suitable. Promoter regions may also further comprise chimeric combinations with each other and with further enhancer regions.

Chimeric genes may optionally comprise a region, immediately preceding the DNA sequence encoding the enzyme involved in the conversion of non-phytotoxic substance to phytotoxin, which encodes a peptide sequence capable of targeting the said enzyme to subcellular organelles such as the chloroplast, peroxisome (other than when the phytotoxin is a peroxide or super oxide anion) or mitochondria and the said targeting protein may have the sequence of (i) a chloroplast transit peptide or (ii) a chloroplast transit peptide-N-terminal portion of a chloroplast protein - chloroplast transit peptide. In particular, for targeting to the mitochondrion, the said region of DNA which immediately precedes the enzyme-coding DNA sequence, encodes a mitochondrial transit peptide sequence. In certain embodiments the transit peptide sequence may be selected from the group consisting of the endogenous transit peptide sequences of the beta-subunit of Nicotinia plumbaginifolia mitochondrial ATP synthase, mitochondria-specific NADP-dependent isocitrate dehydrogenase, NADPH-binding subunit of respiratory chain complex I and yeast mitochondrial tryptophanyl-tRNA-synthetase.

Polynucleotides for use in the present inventive method may comprise one or more chimeric genes which encode enzymes which catalyse reactions involved in the generation of phytotoxins from non-phytotoxic substances. Optionally such polynucleotides comprise yet further genes and chimeric genes, such as a chimeric marker gene. A chimeric marker gene as used herein comprises a marker DNA under expression control of a promoter which is active in plant cells. The marker DNA encodes an RNA, protein or polypeptide which, when expressed in a plant, plant tissue or plant cell allows such plant material to be distinguished from plant material not expressing the marker DNA. Examples of marker genes are genes that provide a specific colour to a cell such as the A1 gene (Meyer et al. (1987) Nature 330, 667) or genes that render plant cells resistant to otherwise lethal selection with antibiotics (e.g. the aac(6') gene encoding resistance to gentamycin, WO 94/01560 or hygromycin phosphotransferase genes providing resistance to hygromycin) or herbicides such as glyphosate (e.g EPSPS genes such as in USP 5510471 or WO 00/66748), phenmedipham (e.g. pmph gene USP 5347047; USP 5543306), bromoxynyl (e.g. genes described in USP 4810648) sulphonylureas (e.g. genes described in EP 0360750), dalapon (genes described in WO 99/48023), cyanamide (genes described in WO 98/48023; WO 98/56238) and genes encoding resistance to glutamine synthetase inhibitors such as L-phosphinothricin (such as, for example, N-acetyl-transferase genes described in EP 0242246, EP 0242246 and EP 0257542). In a preferred embodiment of the polynucleotide of the current invention which comprises a herbicide resistance gene as a marker gene, the said herbicide is a herbicide which is useful for weed control in the crop and, additionally, the herbicide resistance gene is expressed sufficiently to provide robust tolerance to field rates of the said herbicide. In a further preferred embodiment the herbicide is glyphosate and the herbicide resistance gene is an EPSP synthase. However the marker gene may be a gene that provides for positive selection wherein the marker gene encodes an enzyme which provides, in the context of a particular medium, the transformed plant cells with a positive metabolic advantage. USP 5767378 describes a number of suitable positive selection systems and genes.

Where the polynucleotide of the current invention comprises a herbicide resistance gene the herbicide is exogenously applied to crop plants which are interplanted at a sufficient density to eliminate the production of non-hybrid seed originating from non-transgenic self-fertile parent plants. In a preferred embodiment the herbicide is glyphosate or an agronomically useful salt thereof and the said herbicide resistance marker gene is selected from amongst those glyphosate resistance conferring genes described in WO 00166748.

Where a marker gene is present, means for the removal of said marker gene may also be provided. This is desirable where, for example, it is decided to combine traits. In addition it is also desirable to remove herbicide-resistance marker genes which could interfere with the operation of the pro-herbicide-dependent conditional fertility mechanism of the present invention. For example, it might be desirable to remove a phosphinothricin N-acetyl transferase (PAT) herbicide-resistance marker gene from a polynucleotide also comprising a chimeric gene, useful for providing conditional male or female sterility dependent on the exogenous application of D-phosphinothricin pro-herbicide. The presence of the PAT gene could potentially interfere with successful conditional sterility by inactivating the L-phosphinothricin phytotoxin. Thus, polynucleotides which comprise marker genes may optionally comprise specific recognition sites for specific recombinases in positions which flank the marker gene and which allow the sequence to be 'kicked out'. Crossing of a plant carrying the so-flanked marker gene with a plant carrying a gene which encodes the corresponding specific recombinase results in progeny plants from which the marker is specifically excised. Examples of suitable such site-specific homologous recombination systems are the flp/ frt system (Lyznik et al., (1996), Nucleic Acids Res. 24, 3784-3789) and the Cre/Lox system (Bayley, C.C. et al., (1992) PMB, 18, 353-361).

Polynucleotides used in the present inventive method may optionally comprise one or more translational enhancers located within the non translated regions 5' of the protein- encoding sequences. The skilled man is aware of the identity of such suitable translational enhancers - such as the Omega and Omega prime sequences derived from TMV and that derived from the tobacco etch virus, and how such translational enhancers can be introduced into the polynucleotide so as to provide for the desired result of increased protein expression. Further examples include translational enhancers derived from maize chlorotic mottle virus and alfalfa mosaic virus (Gallie et al., (1987) Nucl. Acids Res., 15, 8693-8711; Skuzeski et al., (1990) PMB., 15, 65-79). To further optimise expression of proteins from chimeric genes and chimeric marker genes the said polynucleotides may also further comprise elements such as enhancers, scaffold or matrix attachment regions (SARS or MARS) and introns. Various intron sequences such as the maize adh1 intron 1 have been shown to enhance expression when included into the 5' untranslated region of genes and, optionally, are used in the chimeric genes of the current invention.

Plants which have been transformed according to the invention so as to exhibit the desired male/female sterility characteristics may also have been transformed with a polynucleotide which comprises regions encoding proteins capable of conferring upon plant material containing it at least one of the following agronomically desirable traits: resistance to insects, fungi, viruses, bacteria, nematodes, stress, dessication, and herbicides.

Herbicide resistance conferring genes may, for example, be selected from the group encoding the following proteins: glyphosate oxidase (GOX), EPSP synthase, phosphinothricin acetyl transferase (PAT), hydroxyphenyl pyruvate dioxygenase (HPPD), glutathione S-transferase (GST), cytochrome P450, Acetyl-CoA carboxylase (ACCase), Acetolactate synthase (ALS), protoporphyrinogen oxidase (PPO), dihydropteroate synthase, polyamine transport proteins, superoxide dismutase (SOD), bromoxynil nitrilase, phytoene desaturase (PDS), the product of the *tfd*A gene obtainable from *Alcaligenes eutrophus,* and known mutagenised or otherwise modified variants of the said proteins. The skilled man will recognise the need to close such genes, and the promoters which drive their expression, carefully, having regard to the nature of the enzyme he uses to convert the non-phytoxin substance. In the case that the polynucleotide provides for multiple herbicide resistance such herbicides may be selected from the group consisting of a dinitroaniline herbicide, triazolo-pyrimidines, a uracil, a phenylurea, a triketone, an isoxazole, an acetanilide, an oxadiazole, a triazinone, a sulfonanilide, an amide, an anilide, an isoxaflutole, a flurochloridone, a norflurazon, and a triazolinone type herbicide and the post-emergence herbicide is selected from the group consisting of glyphosate and salts thereof, glufosinate, asulam, bentazon, bialaphos, bromacil, sethoxydim or another cyclohexanedione, dicamba, fosamine, flupoxam, phenoxy propionate, quizalofop or another aryloxy-phenoxypropanoate, picloram, fluormetron, butafenacil, atrazine or another triazine, metribuzin, chlorimuron, chlorsulfuron, flumetsulam, halosulfuron, sulfometron, imazaquin, imazethapyr, isoxaben, imazamox, metosulam, pyrithrobac, rimsulfuron, bensulfuron, nicosulfuron, fomesafen, fluroglycofen, KIH9201, ET751, carfentrazone, mesotrione, sulcotrione, paraquat, diquat, bromoxynil and fenoxaprop.

In the case that the polynucleotide comprises sequences encoding insecticidal proteins, these proteins may be selected from the group consisting of crystal toxins derived from Bt, including secreted Bt toxins such as those known as "VIP"; protease inhibitors, lectins and Xenhorabdus/Photorhabdus toxins. The fungus resistance conferring genes may be selected from the group consisting of those encoding known AFPs, defensins, chitinases, glucanases, and Avr-Cf9. Particularly preferred, insecticidal proteins are cryIAc, cryIAb, cry3A, Vip 1A, Vip 1B, Vip3A, Vip3B, cysteine protease inhibitors, and snowdrop lectin. In the case that the polynucleotide comprises bacterial resistance conferring genes these may be selected from the group consisting of those encoding cecropins and techyplesin and analogues thereof. Virus resistance conferring genes may be selected from the group consisting of those encoding virus coat proteins, movement proteins, viral replicases, and anti-sense and ribozyme sequences which are known to provide for virus resistance; whereas the stress, salt, and drought resistance conferring genes may be selected from those that encode Glutathione-S-transferase and peroxidase, the sequence which constitutes the known CBF1 regulatory sequence and genes which are known to provide for accumulation of trehalose.

Polynucleotides used in accordance with the present invention may have been "modified" to enhance expression of the protein encoding sequences comprised by them, in that mRNA instability motifs and/or fortuitous splice regions may have been removed, or crop preferred codons may have been used so that expression of the thus modified polynucleotide in a plant yields substantially similar protein having a substantially similar activity/function to that obtained by expression of the protein encoding regions of the unmodified polynucleotide in the organism in which such regions of the unmodified polynucleotide are endogenous. The degree of identity between the modified polynucleotide and a polynucleotide endogenously contained within the said plant and encoding substantially the same protein may be such as to prevent co-suppression between the modified and endogenous sequences. In this case the degree of identity between the sequences should preferably be less than about 70%. In addition the sequence around a translational start position may be modified such that it is "Kozack preferred". What is meant by this is well known to the skilled man.

The invention still further includes morphologically normal conditionally fertile whole plants which result from the crossing of plants which have been regenerated from material which has been transformed with the nucleic acid in accordance with the present invention and which therefore provides for such a trait. The invention also includes progeny of the resultant plants, their seeds and parts.

Plants of the invention may be selected from the group consisting of field crops, fruits and vegetables such as canola, sunflower, tobacco, sugar beet, cotton, maize, wheat, barley, rice, sorghum, mangel worzels, tomato, mango, peach, apple, pear, strawberry, banana, melon, potato, carrot, lettuce, cabbage, onion, soya spp, sugar cane, pea, field beans, poplar, grape, citrus, alfalfa, rye, oats, turf and forage grasses, flax and oilseed rape, and nut producing plants insofar as they are not already specifically mentioned, their progeny, seeds and parts.

Particularly preferred such plants include wheat, barley, oats, rice, maize, millet and sorghum.

A preferred method of producing hybrid wheat seed comprises the steps of
(i) transforming plant material with a polynucleotide or vector which comprises a gene conferring male sterility conditional upon exogenous application of a pro-herbicide or other non-phytotoxic substance;
(ii) selecting the thus transformed material; and
(iii) regenerating the thus selected material into morphologically normal conditionally male-sterile whole plants.
(iv) breeding a homozygous conditionally male-sterile female parent line
(v) transforming plant material with a polynucleotide or vector which comprises a gene conferring female sterility conditional upon exogenous application of the same pro-herbicide or non-phytotoxic substance as in (i);
(vi) selecting the thus transformed material; and
(vii) regenerating the thus selected material into morphologically normal conditionally female-sterile whole plants
(viii) Breeding a homozygous conditionally female-sterile male parent line
(ix) Interplanting said conditionally-sterile male and female parent lines at such a ratio as to ensure efficient pollination
(x) Applying said pro-herbicide or other non-phytotoxic substance to the • interplanted parent lines at such a dose and stage in development as to minimise self-fertilisation
(xi) Harvesting hybrid wheat seed from the interplanted parent plants

Also disclosed is a diagnostic kit comprising means for detecting the proteins, or DNA sequences encoding them, which are present in plants produced in accordance with the present inventive method and therefore suitable for identifying tissues or samples which contain these. The DNA sequences can be detected by PCR amplification as is known to the skilled man - based on primers which he can easily derive from the enzyme encoding sequences which are disclosed or mentioned in this application. The enzymes *per se* can be detected by, for example, the use of antibodies which have been raised against them for diagnostically distinguishing the antigenic regions which they contain.

Enantiomerically pure D-Phosphinothricin (D-PPT) may be produced by a method comprising the steps of:
(a) Providing cells which contain an enzyme capable of selectively N-acylating PPT;
(b) Growing said cells in a medium which contains D-L PTT to produce conditioned medium;
(c) Separating the cells from the conditioned medium of (b);
(d) Optionally extracting the conditioned medium with a non-aqueous, non miscible solvent, at various pHs, so that the PPT containing fraction is separated from the fraction that contains molecules more water soluble than is PPT;
(e) Optionally admixing with the conditioned or PPT-containing extracted media of step (d) a cation exchange resin in its protonated form, in an amount, and at pH, sufficient to absorb a substantial proportion of the cations - other than PTT, from the medium;
(f) Admixing with the conditioned medium, extracted medium or medium to result from step (e) a cation exchange resin in its protonated form, in an amount, and at a pH, sufficient to bind the bulk of the PPT in the medium;
(g) Harvesting the cation ion exchange resin from step (f) to which the PPT is bound and selectively eluting PPT from it using an eluting medium having a sufficient pH and ionic strength, with the proviso that the pH of the said eluting medium is not so low as to cause racemisation of the thus eluted PPT.

In respect of the transformation of plant material, those skilled in the art will recognise that although particular types of target material (e.g. embryogenic cell suspension culture or de-differentiating immature embryos) and particular methods of transformation (e.g. using Agrobacterium or particle bombardment) are specified in the examples below, the present invention is not limited to these particular embodiments and such target materials and methods may be used interchangeably. Furthermore the term "plant cells" as used throughout this description of the invention can refer to isolated cells, including suspension cultures as well as to cells in an intact or partly intact tissue such as embryo, scutella, microspore, microspore-derived embryo or somatic cells from plant organs. Similarly, although the specific examples are limited to maize and wheat, the invention is equally applicable to a broad range of agricultural crops which can be transformed using suitable methods of plant cell transformation.

Disclosed are mutant forms of D-amino acid oxidase enzymes and genes which encode them wherein a lysine is present at the position corresponding to the phenylalanine at position 58 of wild-type Rhodotorula gracilis D-amino acid oxidase. Disclosed is a double mutant form of Rhodotorula gracilis D-amino acid oxidase having a lysine at position 58 (F58K) and a serine at position 213 (M213S). In a preferred embodiment the present invention provides a double mutant form of Rhodotorula gracilis D-amino acid oxidase having a lysine at position 58 (F58K) and a threonine at position 213 (M213T). These enzymes are capable of efficiently oxidising D-phosphinothricin and other similar negatively charged D-amino acids such as aspartate and glutamate.

These enzymes and the genes that encode them are used in further applications than in the generation of hybrid crops and, for example,
1) The enzymes may be used in Detection devices for D-amino acids such as D-phosphinothricin (for example as a means of detecting pesticide residues). For example the reduction of oxygen and generation of peroxide ions may be coupled to a range of chemical or electrochemical detection methods and used in a sensor device.
2) The enzymes may be used in Biocatalytic methods for the enantioresolution of DL mixtures of acidic amino acids. For example, the herbicide, phosphinothricin is normally manufactured as the DL racemate whereas only the L form is the active herbicide. It would be desirable to convert all of the D to the L form to achieve a herbicide formulation more pure and twice as active per weight of chemical. The genes and enzymes of the current invention provide a method to achieve this. For example, racemic DL phosphinothricin is added to the growth medium of a host cell such as *E. coli* or yeast etc. transformed to express the F58K, M213S or the F58K, M213T R. gracilis D-amino acid oxidase. Optionally, the host cells are also engineered to express high-levels of L-glutamate aminoacid transferase. The growth medium preferably contains a source of glutamine so that the cells can still grow despite inhibition of glutamine synthetase by the L component of the phosphinithricin. After a suitable time it is found that the racemic phosphinothricin in the medium is substantially all converted to the L form. The medium is then taken to provide substantially pure L-phosphinothricin. Analagous methods which will be obvious to the skilled man may, optionally, use isolated enzymes rather than cell culture methods and, optionally, may use chromatography to isolate the 2-keto acid product from residual L-phosphinothricin. These methods may equally be applied to the enantioresolution of a range of acidic amino acids.

The present invention will be further apparent from the following non-limiting examples taken in conjunction with the associated Sequence Listing and Drawings.

SEQ ID NO: 1 and 2 depict the PCR primers used to obtain the TA29 promoter region.

SEQ ID NO: 3 depicts a DNA sequence, isolated from *Rhodotorula gracilis* which encodes an enzyme having the activity of a D-amino acid oxidase.

SEQ ID NO: 4 and 5 depict degenerate oligos used to provide variant D-amino oxidase.

SEQ ID NO: 6 and 7 depict motifs where alternative amino acids may be substituted in order to provide variant D-amino acid oxidases.

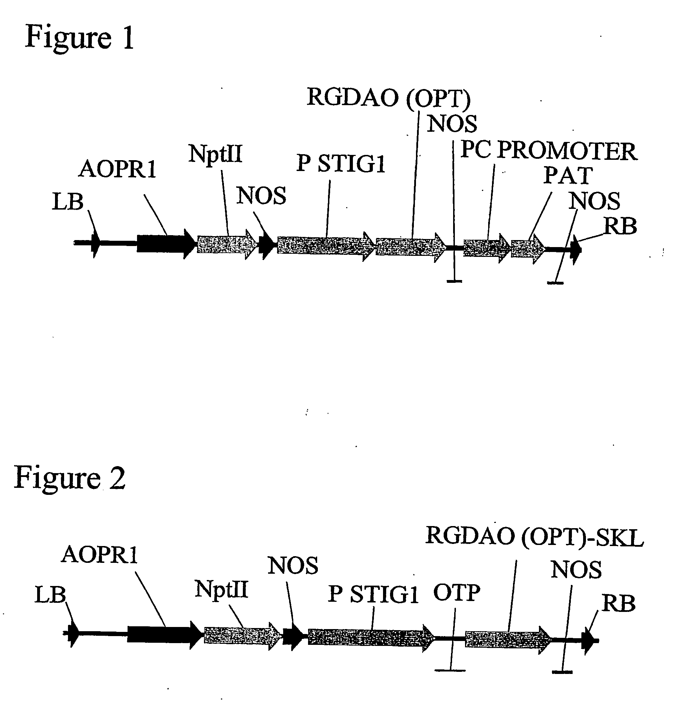

Figure 1 is a schematic representation of a construct for tobacco transformation having Rhodotorula D-amino acid oxidase under operable control of the stig 1 promoter region. The components indicated are LB (left border sequence), AOPR1 (AoPR1 promoter), PSTIG1 (EMBL accession no. X77823), RGDAO (OPT) (SEQ ID NO: 7), PC PROMOTER (EMBL accession no. X16082), PAT (EMBL accession no. A02774), NOS (nos terminator obtained from EMBL accession no. ATU237588) and RB (right border sequence).

Figure 2 is a schematic representation of a construct for tobacco transformation where the Rhodotorula D-amino acid oxidase coding sequence is truncated by 3 codons at the 3' terminus and, at the 5' terminus (RGDAO (OPT)-SKL), is fused to a region encoding an optimised transit peptide (FR2673643).

General molecular biology methods are carried out according to well established methods.

For the most part the following examples each comprise multiple exemplifications of the current invention. Where the term 'promoter region of a gene' is used this is taken to mean DNA sequences which comprise the promoter, sequences upstream of the promoter and also, optionally, all or part of the DNA sequence encoding the 5' untranslated leader region of the mRNA.

### Example 1. Tobacco plants which are conditionally female sterile dependent upon exogenous application of D-phosphinothricin or D-aspartate or D-glutamate

The DNA sequence encoding the D-amino acid oxidase protein sequence P80324 (Swissprot) within the EMBL sequence A56901 is either obtained by RT-PCR from Rhodosporidium toruloides (Rhodotorula gracilis) mRNA or a similar one is obtained synthetically (which makes it easier to control which internal restriction enzyme sites are present and to create flanking sites to facilitate cloning) as, for example, SEQ ID NO: 3 which is designed to account for plant (in this case wheat) codon usage and to minimise DNA features potentially inimicable to expression. The DNA sequence is altered by PCR mutagenesis so that it encodes a mutant form of D-amino acid oxidase having a lysine at position 58 rather than a phenylalanine (F58K) and, optionally, a serine or threonine at position 213 rather than a methionine (M213S or M213T). Flanking PCR-primer and synthetic DNA sequences are designed to place useful unique restriction sites for subcloning. Preferably and in the case where the oxidase coding sequence does not contain confounding internal sites, an Nco1 or Nde1 site is placed at the 5' end to facilitate the cloning of in-frame fusions with sequences added 5' to the ORF such as chloroplast transit peptide encoding sequences. In some variants of the example the D-amino acid oxidase gene is cloned in such a way that the terminal 3 amino acids are truncated and the encoded enzyme is therefore no longer peroxisomally targeted. In an additional series of variants of the method the gene is engineered by PCR so as to encode the Rhodotorula gracilis D-amino acid oxidase with alternative amino acids at positions 213, 223 and 238 and, in particular where, at position 213, the wild type methionine is replaced by His, Thr, Gly, Pro, Gln, Ser, Cys, Asn or Ala, and/or the wild-type tyrosine at position 223 is replaced by His, Thr, Gly, Pro, Gln, Ser, Cys, Asn or Ala and/or the wild type tyrosine at position 238 is replaced by His, Thr, Gly, Pro, Gln, Ser, Cys, Asn or Ala. The methionine at the '213' position is identified as the M in the native protein sequence motif RCTMDSS. The tyrosine at position 238 is identified as the 'Y' within the native protein sequence motif GGTYGVG.

Optionally, restriction sites are placed upstream of the ATG translational start site intervening sequences to conform to plant translational concensus sequences such as according to Kozack.

The 'delta S 13 promoter is a promoter region useful for obtaining preferential expression in female flower parts. This comprises a region -339 to -79 from the SLG13 promoter region fused to the -46 to +8 of the CMV 35S core promoter (Dzelkalns et al (1993) Plant Cell, 5, 833-863). This S13 promoter region is cloned into bluescript sk which plasmid is then further restricted and ligated with restriction fragments comprising the nos 3' transcriptional terminator region and one or other of the amino acid oxidase coding sequences so as to create a 'delta S13-D-amino acid oxidase-Nos terminator' expression cassette within a bluescript sk plasmid. This is then suitably restricted out as, for example, an EcoR1 fragment and, as such ligated back into a suitable site in a vector such as pBIN19 (Bevan (1984) Nucleic Acids Res.) or pCIB200 or pCIB2001 (WO 98/39462) for use for transformation using *Agrobacterium*. As described in WO 98/39462 pCIB200 contains the following unique polylinker restriction sites: EcoR1, Sst1, Kpn1, BglII, Xba1 and SalI. PCIB2001 contains an insertion in the polylinker which adds further unique restriction sites including MluI, BclI, AvrII, ApaI, HpaI and StuI. PCIB200 and pCIB2001 also provides selectable marker genes for plant and bacterial selection on kanamycin, left and right T-DNA borders, the RK2-derived trfA function for mobilization between *E. coli* and other hosts and the oriT and oriV functions from RK2. Alternatively the binary vector pCIB 10 which incorporates sequences from the wide host range plasmid,pRK252 is used (Rothstein et al (1987) Gene 53, 153-161) or one of its derivatives which incorporates both kanamycin resistance genes and the hygromycin phosphotransferase, gene such as pCIB715 is used (Gritz et al (1983) Gene 25, 179-188).

Alternatively the ~ 1.6 kb Stig1 promoter region (derived from EMBL accession X77823) is used. For example the coding region of the GUS gene in the stig1-GUS construct described by Goldman et al (1994) in EMBO J., 13, 2976-2984, is replaced with the DNA sequence encoding either the P80324 or Q99042 coding sequences using suitable restriction enzymes and the resultant stig1-D-amino acid oxidase expression construct cloned into in a suitable vector such as pCIB200 at a position upstream of a 3' terminator sequence adjacent to a suitable marker gene and between T-DNA border sequences.

In a further particular example the T-DNA insert within the binary vector is constructed according to Figure 1. A construct comprising the synthetic DNA sequence (SEQ ID NO:3) encoding Rhodotorula gracilis D-amino acid oxidase altered by PCR mutagenesis so that it encodes a mutant form of D-amino acid oxidase having a lysine at position 58 rather than a phenylalanine (F58K) and, a serine or threonine at position 213 rather than a methionine (M213S or M213T) under operable control of the stig1 promoter region and also the DNA sequence (A02774) encoding L-phosphinothricin N-acetyl transferase (PAT) under operable control of the pea plastocyanin promoter region is cloned into a site between the LB/ npt II gene and the RB of the T-DNA of the binary vector. In brief, the altered SEQ ID NO: 3 encoding the double (F58K, M213S or F58K, M213T) mutant is cloned into plasmid pFse4-Stiglnos (described in WO 99/42598) behind the Stig1 promoter and in front of the nos terminator region (comprised within EMBL: ATU237588) as an NcoI/PstI fragment. The pea plastocyanin promoter region (derived from EMBL Accession number X16082) is obtained from pea genomic DNA by PCR and cloned in front of the PAT gene/nos terminator, The resultant PC-PAT-nos cassette is cloned behind the Stig1-RGDAMOX-nos as a NotI fragment and this whole two gene construct is transferred to a binary vector (pVB6, a Bin19 derivative) as an FseI fragment.

In a further variant of the method the construct used is according to the schematic representation in Figure 2. The Rhodotorula D-amino acid oxidase coding sequence, SEQ ID NO: 3, again site-directed mutated to encode the F58K, M213S or F58K, M213T double mutant form of the enzyme, is truncated by 3 codons at the 3' terminus and, at the 5' terminus, is cloned to place it immediately downstream of a region encoding a chloroplast transit peptide so that a chloroplast transit peptide/ D-amino acid oxidase fusion protein is encoded. The chloroplast transit peptide encoding sequence is derived from the Arabidopsis gene encoding the small subunit of EPSP synthase (Klee et al 1987 in Mol.Gen.Genet., 210, 437). Optionally this is modified to include an Sph1 site at the CTP processing site thereby replacing the Glu-Lys at this location with Cys-Met (SEQ in Fig 9. of WO 92/044490). Correspondingly, an SPh 1 site may be engineered at the N-terminus of the D-amino acid oxidase coding sequence (converting the amino acid following the methionine to a leu). Alternatively the chloroplast transit peptide encoding sequence is derived from the Petunia gene encoding EPSP synthase (Fig. 11 of WO 92/044490). Alternatively-the chloroplast coding sequence is any one of a large number of possibilities including those derived from genes encoding the small subunit of Rubisco and including the so-called 'optimized' chimeric transit peptide sequence (FR 2673643). In all cases, rather than rely on subcloning, the whole desired DNA sequence encoding the chloroplast transit peptide/ double-mutant Rhodotorula D-amino acid oxidase fusion polypeptide may simply be obtained synthetically. This sequence is cloned into a site downstream of the stig1 promoter region and upstream of an (e.g nos) terminator sequence within a suitable vector (e.g. replacing the GUS coding sequence in the vector containing the stig1→ GUS construct described by Goldman et al (1994) in EMBO J., 13, 2976-2984). The whole gene expression construct is then cloned into a suitable site between the right and left borders of the T-DNA of a PVB6 vector.

Tobacco leaf discs are transformed with the recombinant binary vectors using methods similar to those described in Horsch et al (1985) Science, 227, 1229-1231. Many variations of the method may be used. The binary vector can be transformed into, for example, *Agrobacterium tumefaciens* strain LBA 4404 using the freeze thaw method of transformation. Tobacco transformation and whole plant regeneration is performed using *Nicotiana tabacum* var. Samsun according to protocols described by Draper et al (Plant Genetic Transformation, Blackwell Sci. Pub. 1989). Transformation events are selected on MS-media containing kanamycin or other suitable antibiotic. The presence of integrated transgenes is confirmed by PCR. Plants are regenerated and allowed to reach maturity and selfed on to produce seed. Northern and/or Western analysis is used to confirm tissue- specific expression of the D-amino acid oxidase genes. The selected plants are self-fertile but have the condition of conditional female sterility. Seeds of the T1 generation are planted out. Once plantlets have grown to a sufficient size they are tested by PCR for the presence of transgene. PCR positive plants are transferred to the greenhouse. These plants are fully fertile in the absence of exogenously applied proherbicide. A subset of these (putatively) conditionally sterile plants are treated with D-phosphinothricin or D-aspartate or D-glutamate in various amounts and at varying growth stages. Such treatments are carried out on the T1 plants confirmed as PCR positive for the D-amino acid oxidase gene, or, equally, such treatments are carried out directly on plants of the To generation (which are vegetatively cloned so that untreated clones of each event may be-set aside for seed production). The observed fertility is then used as a basis to select suitable plant lines exhibiting the clearest conditional sterility phenotype. For example these amino acids are pure D enantiomers or, alternatively, are DL racemates. For example, they are applied as a foliar spray, prior to or during the early stages of flower formation, at rates usually between 0.25 and 20 kg/ ha. Amino acids which may crystallise out of solution on the leaves following foliar application may be redissolved and remobilised for leaf uptake by further applications of water as a spray mist. Amino acids are, for example, also applied as a root drench and optionally, further applied as - 50 ul of a 10-200 mM solution flooded directly into the buds of emerging florets. Pollen from the treated plants is collected and viability is tested. Plants are obtained which produce relatively little or no seed after treatment with D-phosphinothricin or D-aspartate or D-glutamate but which, nevertheless, under the same treatment conditions do produce near normal levels of viable pollen. Controls include both transgenic and non-transgenic plants and are grown under identical conditions and under an identical regime of physical treatments except that treatment solutions are either water or an equivalent concentration of pure L-amino acid.

In one variant of the method, the amino acid applied is racemic DL phosphinothricin. In this case, the DNA construct used for transformation comprises, in addition to the DNA sequence encoding a D-amino acid oxidase under operable expression control of a tissue specific female floral promoter region such as 'stig 1', also a DNA sequence (EMBL: A02774) a 'PAT' gene under operable control of a promoter region such as the region 5' of the translational start of the plastocyanin gene of the *Pisum sativum* plastocyanin gene (EMBL accession number X16082). For example; the construct is the same as depicted in Fig 1.

The plastocyanin promoter region provides for preferential expression in the green tissues of the plant. It is found, unexpectedly, that such a promoter which, unlike for example, the 35S promoter region, is substantially expressed only in certain tissues of the plant and most notably in green tissues, does, nevertheless, when used in combination with the PAT gene provide for substantially complete reproductive tolerance to the herbicide DL PPT even at rates in excess of 2 kg/ ha. Furthermore, in the absence of the heterologous D-amino acid oxidase being co-expressed in the floral tissues, the plastocyanin/ PAT gene combination provides essentially complete reproductive tolerance with no significant loss of yield despite the PAT expression level being low or non-existent in many of the critical floral tissues when expressed under control of this promoter region. Thus, in this variant of the example, the non-phytotoxic substance D-phosphinothricin is applied in its least costly and most readily available form as the commercial herbicide DL phosphinothricin racemate. At appropriate spray timings and rates between 250 g/ ha and 5 kg/ ha of DL phosphinothricin the treated plants are not visibly damaged but are rendered conditionally female sterile whilst remaining of normal or near-normal male fertility.

### Example 2. Tobacco plants which are conditionally male sterile dependent upon exogenous application of D-phosphinothricin or D-aspartate or D-glutamate

Mutant D-amino acid oxidase protein sequences and the DNA sequences encoding them are as in the preceding example, Example 1.

The TA29 promoter region (Kriete et al (1996) Plant J., 9, 808-818) is cloned from tobacco genomic DNA by PCR using the primers 5'-AACTGCAGCTTTTTGGTTAGCGAATGC-3' (SEQ ID NO: 1) and 5'-CAGACTAGTTTTAGCTAATTTCTTTAAGTAAAAAC-3' (SEQ ID NO: 2). Through a series of restriction and subcloning steps the PCR fragment so obtained is placed upstream of the D-amino acid oxidase coding sequence and a nos transcriptional terminator is added 3' of the coding region. The resultant TA29-D-amino acid oxidase -nos terminator expression cassette is then cloned, obtained as as a suitable restriction fragment and cloned into a binary vector as in Example 1.

Alternatively, any of the above D-amino acid oxidase coding sequence regions are cloned as a suitable restriction fragment (for example BamH1, Bgl/II where synthetic variants of coding sequences are designed so as to remove internal restriction sites) and fused to the CaMV 35S promoter and the nopaline synthase terminator regions by insertion into (for example) the BamH1 site of the binary vector pROK1 (Baulcombe et al (1986) Nature, 321, 446-449) in a sense configuration. The EcoR1-BamH1 fragment carrying the 35S promoter region is then excised and replaced with an EcoR1-BamH1 fragment from pAP30 (Kriete et al (1996) The Plant Journal 9, 809-818) carrying the TA29s promoter region fragment (-810 to + 54). The resultant vectors can be termed pGKTA29_Q99042, pGKTA29_P80324, pGKTA29_Q9HGY3 and pGKTA29_P24552 etc. according to the protein sequence encoded.

Tobacco plant material is transformed, via *Agrobacterium*, with vector and transgenic plants are regenerated in a similar manner to that described in the previous example. The plants produced are self-fertile but are conditionally male sterile. Seeds of the T1 generation are planted but into soil. Once plantlets have grown to a sufficient size they are tested by PCR for the presence of transgene. PCR positive plants are transferred to the greenhouse. These plants are fully fertile in the absence of exogenously applied proherbicide. A subset of these putatively conditionally sterile T1 plants, or, alternatively plantlets of T0 'events' (direct regenerants from transformation) are treated with D-phosphinothricin or D-aspattate or D-glutamate in various amounts and at varying growth stages. Where To plants are treated they are vegetatively cloned so that untreated siblings of the events are set aside for seed production. The observed fertility is then used as a basis to select suitable plant lines exhibiting the clearest conditional sterility phenotype. For example these amino acids are pure D enantiomers or, alternatively, are DL racemates. For example, they are applied as a foliar spray, prior to or during the early stages of flower formation, at rites usually between 0.25 and 20 kg/ ha. Amino acids which may crystallise out of solution on the leaves following foliar application may be redissolved and remobilised for leaf uptake by further applications of water as a spray mist. Amino acids are, for example, also applied as a root drench and optionally, further applied as a 10-200 mM solution directly into the buds of emerging florets.

Pollen from the treated plants is collected and viability is tested. Plants are obtained which shed no or relatively little pollen and/or pollen which is not viable. Pollen collected from some of the treated plants is tested and found to be malformed and non-viable. However, such male infertile plants remain female fertile and produce (hybrid) seed when pollinated with pollen collected from other, untreated non-transgenic or conditionally female-sterile tobacco plants. Controls include both transgenic and non-transgenic plants and are grown under identical conditions and under an identical regime of physical treatments except that treatment solutions are either water or an equivalent concentration of pure L-amino acid.

In an alternative embodiment the promoter region used is a 2.2kb region (EMBO reference X57295) from upstream of the tap 1 gene from snapdragon (Spena et al (1992), Theor. Appl. Genet., 84, 520-527).

Analagous to Example 1, in one variant of the example, the amino acid applied is racemic DL phosphinothricin. In this case the DNA construct used for transformation comprises, in addition to the DNA sequence encoding a D-amino acid oxidase under operable expression control of a tissue specific male floral promoter region such as 'TAP1' or 'TA 29', also a DNA sequence encoding a phosphinothricin N-acetyl transferase gene such as the 'PAT' gene under operable control of a promoter region such as that from the plastocyanin gene (in this case the region from the Pisum sativum plastocyanin gene). At appropriate spray timings and rates between 250 g/ ha and 5 kg/ ha ofDL phosphinothricin the treated plants are not visibly damaged but are rendered conditionally male sterile whilst remaining of normal or near-normal female fertility.

### Example 3. Chimeric genes capable of being preferentially expressed in the male reproductive structures of wheat and encoding enzymes capable of oxidising D-phosphinothricin, D-glutamate or D-aspartate.

Mutant D-amino acid oxidase protein sequences and the DNA sequences encoding them are as in Example 1. Plasmid pGK73 carries the TA29s promoter region EcoR1-BamH1 fragment from -810 to +54 (Kriete et al (1996), 9, 809-818). This restriction fragment or a similar suitable PCR-generated fragment is cloned, preferably as an in-frame fusion, at a position upstream of the DNA sequence encoding, for example, the double mutant (F58K, M213S or F58K, M213T) R. gracilis D-amino acid oxidase into bluescript sk. Using a suitable series of restriction, ligation and subcloning steps a nos transcriptional terminator is added 3' of the coding region to generate, according to the coding sequence, alternative expression cassettes of the type TA29-carboxylesterase-nos in Bluescript sk plasmids, pBLTA_RGF58KM213T, pBLTA_RGF58KM213S etc..

In a further example, the anther specific SGB6 promoter region SEQ ID NO: 1 of USP 5470359 is used. For example, pSGBNE1 containing a 3 kb genomic EcoR1-Nhe1 subcloned fragment from pSGB6g1 (USP 5470359) is further subcloned to place a 1558 bp ApaII/Xba1 fragment blunt cloned into bluescript ks at the SmaI site. As before, through further restriction and cloning steps this fragment is fused in frame upstream of a mutant D-amino acid oxidase DNA encoding sequence. Again a nos terminator is added 3' of the coding region to create, alternative, Bluescript sk plasmids, pBLB6_RGF58K etc. comprising the alternative SGB6-DAMOX-nos expression cassettes.

In a similar set of examples the RA8 anther-specific promoter region from rice (EMBL/ genbank accession AF042275; Jeon et al (1999) PMB, 39, 35-44; WO 00/26389) is similarly also fused at a site in-frame and upstream of one or other of the DNA sequences encoding F58K mutant D amino acid oxidase and a nos 3' terminator to comprise alternative RA8-DAMOX-nos expression cassettes in a series of bluescript sk vectors, pBLRA8_RGF58K,M213S etc.

### Example 4. Chimeric genes capable of being preferentially expressed in the female reproductive structures of wheat and encoding enzymes capable of oxidising D-phosphinothricin and/or D-aspartate and/or D-glutamate

DNA sequences encoding D-amino acid oxidase protein sequences are obtained as described in Example 1.

The genomic clone pSH64 was deposited under the terms of the Budapest treaty on 27/02/1998 with NRRL and assigned the number NRRL B-21920. It was detected as a genomic clone hybridising to the silk-specific cDNA clone B20014-2 (WO 98/39462). Chimeric genes which are expressed preferentially in female reproductive structures are constructed as follows. A bluescript ks-derived plasmid similar to pSH70 having an 'empty' expression cassette comprising, from 5' to 3', the B200i 5' promoter region consisting of nucleotides 1-3790 of SEQ ID No 11 of WO 98/39462, a BamH1 site and the B200i 3' untranslated terminator region comprising nucleotides 4427-6397 of sequence ID No. 11 of WO 98/39462 is constructed as described in WO 98/39462. Using a partial BamH1 digestion or, alternatively by further subcloning, PCR and ligation steps alternative D-amino acid oxidase coding sequences are ligated into the position at or adjacent to the BamH1 site such that they are immediately 3' of the B200i promoter region and 5' of the B200i terminator region. Accordingly, a series of bluescript vectors pBLB200_RGF58K, pBLB200_RGF58KM213T, pBLB200_RGF58KM213S etc. encoding the alternative mutant D-amino acid oxidase-B200i expression cassettes are created.

Alternatively, as described in WO 98/39462, a Pst I/ Nco I fragment of the 5' promoter region of the P19 gene is excised from the genomic clone X2-1 which was deposited under the terms of the Budapest treaty on 27/02/1998 at NRRL and assigned accession number B-21919. The Nco I site at nucleotide 1088 of SEQ ID No 14 of WO 98/39462 corresponds with the ATG translational start of the P 19 gene. Using appropriate subcloning, restriction, ligation and PCR steps this fragment is ligated to form a in-frame fusion with one or other of the DNA sequences encoding D-amino acid oxidase and a nos terminator sequence is added 3' of the coding sequence. Accordingly, a series of bluescript vectors pBLP19_RGF58KM213S, pBLP19_RGF58KM213T etc. encoding the alternative P19-D-amino acid oxidase-nos expression cassettes are created. Alternatively, using similar standard methods, similar plasmids are obtained having the 5' promoter region (comprising some or all ofnucleotides 1-3987 of SEQ ID No 2 of WO 98/39462) of the P26 gene in place of the P19 promoter region. The genomic P26-A4 clone, pCIB10302 deposited under the terms of the Budapest Treaty on Jan 21 1997 with the Agricultural Research Service patent culture collection, (NRRL) accession number NRRL B-21655 is subcloned as described in WO 98/39462. Accordingly, a series of bluescript vectors pBLP26_ RGF58KM213T, pBLP26_RGF58KM213S etc. encoding the alternative P19-D-amino acid oxidase-nos expression cassettes are created.

### Example 5. A pair of complementary constructs useful in a method to provide (a) a female inbred parental line which is conditionally male-sterile dependent upon the application of DL phosphinothricin and (b) a complementary male inbred parental line which is conditionally female sterile dependent upon the application of DL phosphinothricin.

The first DNA construct suitable for providing a female inbred parental cereal or rice plant line which is conditionally male-sterile dependent upon the application of DL phosphinothricin comprises three genes A), B) and C). A) consists of a DNA sequence encoding a PAT enzyme capable ofN-acetylating L-phosphinothricin under operable control of the ~ 1kb promoter region from the barley plastocyanin gene (EMBL: Z28347) and a suitable terminator region such as that from the nos or 35S gene, B) consists of a PAT encoding sequence similar to the first but this time under operable control of a tissue specific female floral promoter region (such as P19 or P26 as described above) plus a suitable terminator and C) consists of a suitable DAMOX encoding sequence as described in Examples 1,10 and 11, encoding, for example, a double or triple mutant of the F58K mutant form of *Rhodotorula gracilis* D-amino acid oxidase having changes at positions 213, 223 and 238 and, in particular where, at position 213, the wild type methionine is replaced by His, Thr, Gly, Pro, Gln, Ser, Cys, Asn or Ala, and/or the wild-type tyrosine at position 223 is replaced by His, Thr, Gly, Pro, Gln, Ser, Cys, Asn or Ala and/or the wild type tyrosine at position 238 is replaced by His, Thr, Gly, Pro, Gln, Ser, Cys, Asn or Ala under operable control of a tissue specific male floral promoter region (such as SGB6 or RA8 as described above) and a suitable terminator region. In preferred examples the encoded *R. gracilis* DAMOX enzyme is a double F58K, M213S or F58K, M213T mutant form. This construct is assembled using methods which are standard in the art and informed by the previous examples.

The second DNA construct suitable for providing a male inbred parental cereal or rice plant line which is conditionally female-sterile dependent upon the application of DL phosphinothricin comprises three genes A), D) and F). A) consists of a DNA sequence encoding a PAT enzyme capable ofN-acetylating L-phosphinothricin under operable control of the promoter region from the barley plastocyanin gene and a suitable terminator region such as that from the nos or 35S gene, D) consists of a PAT sequence similar to the first but this time under operable control of *the same* tissue specific male floral promoter region (such as SGB6 or RA8) as used in construct 1 plus a suitable terminator and F) consists of a suitable DAMOX gene as, for example, used in construct 1 and under operable control of *the same* tissue specific female floral promoter region (such as P 19 or P26) as used in construct 1 and a suitable terminator region. This construct is assembled using methods which are standard in the art and informed by the previous examples.

A pair of DNA constructs of this example contain, for example, the following elements

### Construct 1

A = Barley plastocyanin promoter region → PAT encoding sequence, Nos terminator;
B = P26 promoter region → PAT encoding sequence, 35S terminator;
C = RA8 promoter region → Rhodotorula D-amino acid oxidase (F58K,M213T mutant) encoding sequence, Nos terminator

### Construct 2

A = Barley plastocyanin promoter region → PAT encoding sequence, Nos terminator;
D = RA8 promoter region → PAT encoding sequence, 35S terminator;
E = P26 promoter region → Rhodotorula D-amino acid oxidase (F58K, M213T mutant) encoding sequence, Nos terminator

### Example 6. Polynucleotide vectors for transformation of wheat

Examples 3, 4 and 5 describe the construction of various chimeric genes in expression cassettes which are usually cloned into bluescript sk. Optionally these vectors are prepared in bulk for direct DNA transformation for use with a co-bombarded selectable marker such as pSOG35 (DHFR/methotrexate) or pUbi-Hyg (hygromycin phosphotransferase/ hygromycin) as described in WO 98/39462. Preferably, after bulk preparation, the vectors are linearised using a suitable restriction enzyme to remove the ampicillin resistance gene of bluescript.

Optionally, rather than use co-bombardment the said bluescript vectors are further engineered by standard methods so that they further comprise a plant selectable marker gene such as kanamycin resistance, hygromycin resistance, methotrexate resistance or glyphosate resistance gene and are used directly. In some of the foregoing examples a PAT gene is integral to the design of the vector and, in these cases, DL phosphinothricin may optionally be used for selection at some stage after transformation.

Alternatively, expression cassettes are excised within a suitable restriction fragment and cloned into pIGPD9 derived vectors (described in Figure 12 of WO 00/66748). The use of this vector for transformation avoids transfer of antibiotic marker genes to the plant since its maintenance in bacteria relies on complementation of an auxotrophic E.coli mutant. The vector comprises a gene expressing IGPD (the HisB product) and is further engineered to comprise a plant selectable marker gene such as an EPSPS gene cloned into the Xma I site as, for example, in pZEN16i and pZEN18i of WO 00/66748. Alternatively a marker gene which provides positive selection on mannose or xylose is used (USP 5767378).

In particular examples of using pIGPD9 vectors, plasmids for wheat transformation are constructed. Illustrative examples are pZEN18_ BLB200_Q99042 and pZEN18_ BLRAB_Q01470. These are pIGPD9-derived vectors comprising the pZEN18 EPSPS gene (WO 00/66748) and, in this case, either the B200i-)D-amino acid oxidase-B200i or the RA8-D-amino acid oxidase-nos expression cassettes, respectively.

Large-scale DNA preparations for use in plant transformation are obtained using the Maxi-prep procedure (Qiagen) using protocols supplied by the manufacturer.

### Example 7. Transformation/ regeneration of wheat with polynucleotides comprising chimeric genes preferentially expressed in either male or female reproductive structures and which encode enzymes capable of oxidising D-phosphinothricin and/or D-aspartate and/or D-glutamate

In one example, immature embryos (0.75-1.0 mm in length) of genotype UC703 are plated on MS medium containing 3 mg/1.2,4-D and 3% sucrose. After approximately 4h the embryos are plated onto MS medium containing 15% maltose, 3% sucrose and 3 mg/12,4-D overlaid with a filter paper supported slab of agarose containing the same components. The embryos are allowed to plasmolyze for 2-3h before bombardment.

DNA prepared as described in Example 6 and in the foregoing examples is precipitated onto micrometer size gold particles using standard procedures. Four target plates with 16 embryos per target are shot twice with a DuPont Biolistics helium device using a burst pressure of 1100 psi. Plates are shot with an 80 mesh screen in place between the carrier stage and the target. After bombardment targets are placed in the dark at 25°C for 24h before the slabs with the embryos are laid onto plates of MS medium containing 3% sucrose and 3 mg/12,4-D. The individual embryos are removed from the slabs and placed directly on fresh medium of the same composition after another 48h. Approximately 6 weeks after gene delivery the tissue is placed on MS medium with 3 mg/12,4-D, 3% sucrose and 0.2 mg/ 1 of methotrexate for a 3 week period. The tissue is then placed on regeneration medium comprised of MS medium containing 1 mg/ 1 zeatin riboside and 1 mg/l methotrexate. After 2 weeks regenerating plantlets are placed in sterile containers with half- strength MS medium containing 2% sucrose, 1 mg/ 1 napthylacetic acid and 4 mg/l methotrexate.

In particular variants of the example the vectors comprising chimeric genes preferentially expressed in male reproductive structures are co-bombarded with alternative selectable marker genes. Thus, for example, DNA of plasmids is prepared and coated onto gold particles along with pUbiHyg (a plasmid encoding hygromycin phosphotransferase under operable control of the maize polyubiquitin promoter). In this case transformation and regeneration is carried out as described above except that, following bombardment, the regeneration media contain increasing concentrations of hygromycin between 2 and 20 mg/l.

In a further example wheat is transformed with pZEN18_BLB200_RGF58KM213S (D-amino acid oxidase), selected using glyphosate and regenerated as described in Example 15 of WO 00/66748.

DNA is extracted from leaf tissues of plants derived from transformation and PCR is run for the presence of selectable marker gene and the gene encoding D-amino acid oxidase. PCR positive plants are propagated. During flowering, pistils and anthers are collected and RNA is prepared. DNA expression is confirmed by Northern analysis. In addition, D-amino acid oxidase genes are expressed using pET vectors in *E*. *coli* and part purified. The protein bands of the expressed protein is cut out of an SDS gel and used to generate polyclonal antibodies. These antibodies are used to detect expression in flower tissues and other tissues by Western analysis.

### Example 8. A method of efficiently producing hybrid cereal crops wherein DL phosphinothricin is applied both for weed control and at the same time as the chemical hybridising agent and wherein the F1 hybrid generation of plants resulting from the so-produced hybrid seed is both vegetatively and reproductively substantially tolerant to the application of DL phosphinothricin.

Chemical hybridising agents are expensive. It would be desirable to use a relatively cheap substance such as a commercial herbicide as a chemical hybridising agent. This would also achieve further efficiency since weed control could be combined with chemical hybridisation. However there are a number of problems to overcome in order that this proposition be realised. Firstly, male and female parental lines would need to be established which are tolerant to the herbicide in question. Furthermore, in order to achieve the desired 'conditional' fertility in response to application of the herbicide the two lines would need to be engineered in such a way that the tolerance to the herbicide did not extend to all tissues but was expressed in a tissue specific manner so that each one of the required floral tissues remained selectively susceptible. Thus, in one line (the female parent line), the bulk of the plant plus the female tissue must be rendered tolerant whilst some critical part of the male floral tissue must remain susceptible to the application whereas in the other (the male parent line), the converse is needed with only some critical part of the female gamete forming tissue remaining susceptible. Even given that this can be achieved there remains a further problem to overcome in respect of the hybrid seed and F1 generation. Given that this generation of the crop would, necessarily, contain at least two genes capable of conferring resistance to the herbicide it would be desirable that this same herbicide could also be used for weed control in the crop. However, it is very difficult to conceive of a combination of herbicides, tissue specific promoter regions and tolerance genes that would permit this use of the same herbicide in the F1 generation. It would be likely that the hybrid crop would display vegetative tolerance but little or no grain yield after herbicide application to the F1 generation. For example, for the herbicide glyphosate the usual mechanism of resistance is the expression of a resistant form of EPSP synthase. It is difficult to identify a promoter region or combination of promoter regions that would permit sufficient expression of a R-EPSPS in all tissues and at all times *other* than, say, at a critical stage in the development of stamens or stigmas. The most straightforward way around this would be to use an antisense or similar approach wherein expression of the R-EPSPS is driven by a tissue non-specific/constitutive promoter and only *locally and transiently* suppressed in, for example, the stamens due to expression of an antisense EPSPS gene (see for example WO 99/46396). However, in that case the suppression of expression in the stamen (or stigma) would be driven by a *dominant* gene. It is clear that, for any such mechanism, the application of the herbicide to the F1 generation would result in a sterile non-yielding crop due to the additive effects of the dominant male and female conditional sterility genes.

The current invention provides a method of overcoming the problem of enabling the use of a cheap commercial herbicide, DL phosphinothricin, as both weed control and hybridising agent in the production of hybrid cereals and which method, furthermore, provides resultant hybrid cereals or rice in which DL phosphinothricin (or L-phosphinothricin) can be safely used for weed control without substantial loss of yield. As a yet further benefit, selfed seed from the F1 generation which may later arise as volunteers in subsequent crops will be easier to manage since they, themselves, will generally be sterile if sprayed with controlling amounts of DL phosphinothricin. The same holds for the progeny of pollen outcrossing from the F1 plants to weeds (e.g red rice) or other cereals. The current invention provides genes and enzymes that convert a non-phytotoxic component, D-phosphinothricin, of a commercial herbicide formulation DL phosphinothricin, into the active L form. The PAT gene which converts L phosphinothricin to N-acetyl L-phosphinothricin is known already and is used commercially to provide tolerance to DL phosphinothricin in crops. A further critical observation germane to the current example is that, surprisingly, wheat containing a PAT gene under operable expression control of the barley plastocyanin promoter region is found to be substantially reproductively tolerant to the application of DL phosphinothricin at rates up of at least 2kg/ ha. Thus a critical feature of the constructs described in Example 6 which are used to provide the plants of the current example is that the PAT gene which provides the resistance trait is expressed under operable control of a promoter region which provides for expression in substantially *only* the green tissues. A characteristic of such a useful promoter region is that it should express PAT in such a way that it protects adequately all the non-green floral tissues from foliarly applied DL phosphinothricin whilst, at the same time, providing only a minimal level of PAT expression in the floral tissue itself and especially low in those parts targeted for conditional sterility. With PAT expressed under operable control of the barley plastocyanin promoter region this condition appears to be met since substantially all of the L-phosphinothricin which is sprayed enters *via* the leaves and is intercepted and converted to non-phytotoxic N-acetyl-L-phosphinothricin before it is translocated to developing floral tissues. Thus, in the current invention, the L-phosphinothricin which causes the tissue selective sterility effects in the parental lines is only generated transiently and locally from phloem mobile non-phytotoxic D-phosphinothricin *via* D amino acid oxidase. By exactly matching the floral control elements driving expression of PAT to those elements which drive expression of D-amino acid oxidase in the complementary pair of constructs (Example 5) it is ensured that, in the F1 hybrid, the transient burst of L-phosphinothricin in the target floral tissue is rapidly neutralised by a corresponding burst of PAT expression at the same time and in the same local tissue. Thus application of the herbicide induces no sterility effect in the hybrid. However, in further generations, the florally corresponding PAT and D-amino acid oxidase of the hybrid will segregate apart and thus, once again, the resulting plants will be male or female sterile upon application of controlling amounts of DL phosphinothricin.

Using the methods described in Examples 6 and 7, the constructs described in Example 5 are transformed into wheat or (using standard superbinary vector methods) into rice which is selected and regenerated into plantlets. T0 transformant events are selected (using clonal propagation of tillers to maintain untreated lines) and suitable events for breeding on as, alternatively, male inbred parental lines which are conditionally female sterile dependent upon the application of DL phosphinothricin or female inbred lines which are conditionally male sterile dependent upon the application of DL phosphinothricin are selected using methods essentially as described in examples 1 and 2. The best lines exhibit the best herbicide tolerance, minimum yield loss, cleanest conditional sterility phenotype etc. The alternative male parent and female parent lines are selected and, optionally, backcrossed into suitable elite lines for a number of generations. The genetic inserts in these finally selected events are fully characterized as are the genetics of the inheritance of the conditional fertility and herbicide resistance traits and the characteristics of expressed gene products.

The, thus selected, female and male parental lines are then interplanted together in suitable ratios in a field and sprayed with DL phosphinothricin at a suitable rate between 0.05 and 5 kg/ ha and timing up to the period of early flowering selected to optimise the production of hybrid seed. The seed thus produced have the advantage that they will give rise to plants which not only benefit from hybrid vigour but which are also tolerant to the herbicide formulations containing DL phosphinothricin which may thus be used for selective weed control in the crop. The hybrid seed also have the advantage that the herbicide tolerance trait that they express will be only incompletely passed onto future selfed generations or outcrossed into related weeds. Thus, for example, the hybrid rice resulting from this invention can be grown using DL phosphinothricin as weed control agent without significant loss of yield. However future generations of red rice plants which arise as the progeny of pollen from the hybrid rice outcrossing with red rice female parents will be vegetatively tolerant to treatment with DL phosphinothricin but have reduced self-fertility (owing to the expression of a D-amino acid oxidase in the floral tissue) and thus produce little grain. Hence using hybrid rice of the current invention DL phosphinothricin may be used for weed control with much reduced future risk of grain contamination with red rice as a result of the herbicide resistance trait having outcrossed into the closely related red rice. Similarly, second generation volunteers of rice or wheat which arise from the hybrid crop will, for the most part, not produce grain after spraying with DL phosphinothricin.

### Example 9. Transformation/ Regeneration of maize with a polynucleotide comprising a chimeric gene preferentially expressed in male reproductive tissue and which encodes an enzyme capable of oxidising D-phosphinothricin.

RA8-D-amino acid oxidase-nos expression cassettes are cloned into a series of bluescript sk vectors, pBLRA8_RGF58KM213T, pBLRA8_RGF58KM213S etc. as described above. Optionally, these are co-bombarded with DNA comprising selection markers such as pUbiHyg or pSOG35; selected and regenerated using hygromycin or methotrexate as described, for example, in Example 11 of WO 98/39462.

Alternatively, pZEN18_BLRA8_RGF58KM213S etc. are directly bombarded or transferred on silicon carbide whiskers into maize cells and maize plants are selected and regenerated on glyphosate as, for example, described in Examples 12 and 13 of WO 00/66748.

Alternatively, maize transformation is carried out using *Agrobacterium tumefaciens* containing a superbinary vector. For example, the pZEN18 expression cassette and the BLRA8_F58K D-amino acid oxidase chimeric gene is excised from, pZEN18_ BLRA8_RGF58K and cloned into positions between the right and left T-DNA borders of a pSB1-derived superbinary vector through a series of subcloning and homologous recombination in a series of steps similar to those described in WO 00/66748. Plant material derived from immature embryos is infected with *Agrobacterium* containing superbinary vector comprising the glyphosate marker gene and the chimeric gene of the current invention. Plants are selected and regenerated using glyphosate as described in WO 00/66748.

DNA is extracted from leaf tissues of plants derived from transformation and PCR is run for the presence of selectable marker gene and the gene encoding mutant D-amino acid oxidase. PCR positive plants are propagated. During flowering pistils and anthers are collected and RNA is prepared. DNA expression is confirmed by Northern analysis. In addition, mutant D-amino acid oxidase genes are expressed using pET vectors in *E*. *coli* and part purified. The protein band of the expressed protein is cut out of an SDS gel and used to generate polyclonal antibodies. These antibodies are used to detect expression in flower tissues and other tissues by Western analysis.

### Example 10 Site-directed mutagenesis to generate further mutants derived from the F58K mutant form of R.gracilis D-amino acid oxidases with further improved abilities to oxidise D-phosphinothricin and/or D-aspartate etc.

This example concerns the production of genes which encode variants of *R*. *gracilis* D-amino oxidase having improved ability to oxidise D-phosphinothricin and/or other acid-side chain D-amino acids. These genes are used in preferred embodiments of the invention, described in the other examples, where sterility is made conditional upon application of D-phosphinothricin or D-aspartate. In the particular current example these genes encode enzymes having, in addition to the F58K mutation, a single amino acid change at position '213' and/ or at position '238'. The skilled man will recognise that entirely analogous methods are used to effect a similar series of mutations to replace the tryrosine at the equally preferred position 223 with the same set of alternative amino acids. The methionine at the '213' position is identified as the M in the native protein sequence motif RCTMDSS (SEQ ID NO: 6). The tyrosine at position 238 is identified as the 'Y' within the native protein sequence motif GGTYGVG (SEQ ID NO: 7). There are many approaches known in the art to providing a series of genes encoding a series ofD-amino acid oxidase variants with amino acid changes at one or both of these positions. The choice of DNA template for mutagenesis also depends upon the intended use. Thus, for example, where the intended use of the mutant gene is for expression in plants then a plant-optimized synthetic DNA which encodes an *R. gracilis* D-amino acid oxidase such as the F58K mutant encoding mutant form of SEQ ID NO: 3 is a suitable starting point. On the other hand, where the intended immediate use of the mutant gene is as a starting point for further rounds of random mutagenesis and improvement in a yeast- or *E*. *coli*-based selection system (as in Example 11) then the F58K mutant of either the native DNA sequence or a synthetic sequence optimised for expression in *S. cerevisiae* is more suitable.

A preferred method for providing suitable variants of *R*. *gracilis* D-amino acid oxidase is through the use of degenerate oligonucleotides using Strategenes Quickchange mutagenesis kit. Methods used are according to the manufacturers instructions.

For example in the case that the F58K mutant encoding mutant form of native *R. gracilis* DNA sequence encoding D-amino acid oxidase be the template DNA for mutagenesis) pairs of 'top' (RGMUTTOP) and 'bottom' (RGMUTBOT) degenerate oligonucleotides may suitably be of 50 -250 nucleotides in length and designed to comprise, within them, sequence regions as follows.

RGMUTTOP comprises within it a sequence (SEQ ID NO: 4)

RGMUTBOT comprises within it a sequence (SEQ ID NO: 5)

In addition, these two oligonucleotides, RGMUTTOP and RGMUTBOT comprise at each end, sequences which, once the two oligonucleotides are annealed with each other will constitute 5' and 3' ends which will exactly match the ends created when the template DNA is cut at a suitable pair of unique restriction sites (i.e designed so that the annealed oligonucleotides can replace a unique restriction fragment cut out of the D-amino acid oxidase encoding template DNA).

0.5 to 1.0 ug of each oligonucleotide is transferred to a 0.5 ml Eppendorf centrifuge tube and heated at a suitable temperature (e.g 94°C, depending on calculated melting points) for 5 minutes and annealed slowly by cooling to room temperature. Template DNA (for example pYES6/CT yeast shuttle vector) is then cut with two restriction enzymes (according to the two unique restriction sites in the template DNA which span the region including the two codons to be replaced and that characterise the ends of the annealed DNA), gel purified, ligated with the annealed oligonucleotide, and transformed into yeast so that the alternative D-amino acid oxidases created by mutagenesis are expressed. Then, as described, yeast clones which yield the best growth on analogues of D phosphinothricin (such as D-homocysteic acid) or on D-phosphinothricin (when the PAT gene is co-expressed) as sole nitrogen source are selected as those containing the variant D-amino acid oxidase encoding sequences with the desired properties. Alternatively D-amino acid oxidase expression is carried out in some microorganism other than yeast and, for example, under expression control of the T7 promoter of a pET vector in an *E*. *coli* lysogen. In this case, following transformation, individual colonies may be picked, replica plated, grown, induced, lysed and screened for the desired substrate activity versus D-phosphinothricin using methods known in the art (for example, a fluorimetric screen for peroxide generation or a colorimetric assay for ammonia generation or for the 2-keto acid using well established assay methods. The test organism transformant lines may suitably be grown in 2ml wells of microtitre plates, lysed *in situ* and assayed colourimeterically for D-amino acid oxidase activity using, alternatively, phosphinothricin or D-aspartate as substrate (depending upon optimisation of which activity is being sought after). Lines giving the highest levels of activity are selected. Alternatively, the transgenic *E*. *coli* lines are further transformed so that they express the PAT gene (for example, constitutively) and induced with IPTG so that they express the 'test' mutant D-amino acid oxidase. Induced lines which exhibit the best growth on minimal medium provided with phosphinothricin (or, optionally, a phosphinothricin analogue) as the major N source (optionally a small amount of ammonium ions are included) are selected.

Optionally, lines are selected not only on the basis of maximising the ability to utilise D-phosphinothricin or other acid-side chain D alpha amino acids but also to improve thermal stability (e.g extracts or cell lines are subjected to a short heat treatment prior to enzyme assay or cells are grown at raised or lowered temperatures) or other properties.

The yeast or other microbial clones thus selected are grown up, DNA is prepared and the full length D-amino acid oxidase DNA sequence cloned via proof reading PCR and cloning into pCRBlunt II using Invitrogens Zero Blunt TOPO kit. The D-amino acid oxidase encoding sequences characterising the selected clones are determined. These D-amino acid oxidase coding sequences are further subcloned for expression in a pET vector (e.g Novagen pET 24a) and transformed into *E. coli* BL21 DE3. The cells are grown in a fermenter on LCM50 medium containing 100 ug/ ml kanamycin, induced with IPTG, harvested, broken and the extract part-purified and assayed for D-amino acid oxidase activity (as detailed below). D-amino acid oxidase genes are selected which encode D-amino acid oxidase enzymes yielding acceptable stability and the highest activity (kcat/Km) per mg of pure protein versus D-phosphinothricin at pH 7.0.

Additionally a series of particular DNA sequences encoding particularly targeted D-amino acid oxidase enzymes are generated. In particular, genes are generated which encode the F58K mutant form of *Rhodotorula gracilis* D-amino acid oxidase with further mutational changes at positions 213, 223 and 238 and, in particular where, at position 213, the wild type methionine is replaced by His, Lys, Arg, Thr, Gly, Pro, Gln, Ser, Cys, Asn or Ala, and/or the wild-type tyrosine at position 223 is replaced by His, Lys, Arg, Thr, Gly, Pro, Gln, Ser, Cys, Asn or Ala and/or the wild type tyrosine at position 238 is replaced by His, Lys Arg, Thr, Gly, Pro, Gln, Ser, Cys, Asn or Ala. The methods used are the same as described above except that, rather than a mixture of oligonucleotides, individual oligonucleotide pairs are designed and used to effect each single or double amino acid change. Each resulting mutant D-amino acid oxidase coding sequence is cloned for (untagged) expression behind the T7 promoter in Novagen pET 24A and transformed into *E.coli* BL21 DE3. The cells are grown in a 1.01 fermenter in LCM50 medium supplemented with 100 ug/ml kanamycin, induced for expression with 1 mM IPTG and harvested by low-speed centrifugation.

### LCM50 Medium contains (in 1 litre)

KH₂PO₄ (3g), Na₂HPO₄ (6g), NaCl (0.5g), Casein hydrolysate (Oxoid) (2g), (NH₄)₂SO₄ (10g), Yeast Extract (Difco) (10g), Glycerol (35g) (these ingredienst are made up in solution and autoclaved). The following additional ingredients are filter sterilised as solutions and added to the media: MgSO₄ (2.5ml of 246.5mg/ml solution), Thiamine.HCl (1ml of 8mg/ml soln.) CaCl₂.2H₂O (0.2ml of 147g/l solution), *Fe SO₄.7H₂O/Citric acid stock (2ml), **Trace element solution (5ml) and make up to 1 litre.

**Fe SO₄.7H₂O* / *Citric acid stock per 100ml* consists of Fe SO₄.7H₂O (0.415mg), Citric acid (0.202mg):

***The Trace element solution composition per 1ml* is AlCl₃.6H₂O (20mg), CoCl₂.6 H₂O (8mg), KCo(SO₄)₂.12 H₂O (2mg), CuCl₂.H₂O (2mg), H₃BO₃ (1mg), KI (20mg), MnSO₄.H₂O (0.8mg), Na₂MoO₄.2H₂O (4mg), ZnSO₄.7H₂O (4mg)

Approximately 7g wet weight of cells is washed in water. The cells are resuspended in an equal volume of 50 mM /Mops/ KOH buffer at pH 7.0 containing 2 mM EDTA, 2 mM DTT and 0.01 mM FAD. Cells are evenly suspended using a glass homogeniser and then disrupted using a one shot head in the Constant Systems (BudBrooke Rd, Warwick U.K.) Basic Z cell disrupter at 13500 psi. The crude extract is kept cold (∼4°C) centrifuged at 30,000 gav for 1 h and the pellet discarded. Some of the extract protein is run out on an SDS PAGE gel stained with Coomassie Blue and, through side by side comparison with similarly prepared extracts of cells containing only 'empty' pET vector it is estimated that 2-50% of the total soluble protein in the extract is D-amino acid oxidase. Some of the extract protein is exchanged into 50 mM Mops/ KOH buffer at pH 7.0 containing 0.01 mM FAD. This is diluted with the same buffer in a standard oxygen electrode cell (calibrated at 25°C between zero and a saturated concentration of oxygen). Optionally, the D-amino acid oxidase is further purified using ion-exchange, phenyl sepharose, fractional ammonium sulphate precipitation and gel filtration. Assays, at 25°C, are started by addition of a 200 mM solution of the ammonium salt of DL phosphinothricin to the diluted enzyme or, by addition of 25 mM D-aspartate. For measurement of Vmax and Km values, substrate concentrations are varied in the normal way. Vmax values are estimated on the basis of total protein and the estimated purity of the D-amino acid oxidase. Based on SDS PAGE, mutant D-amino acid oxidase normally constituted 15-35% of the soluble protein in crude protein extracts (or greater where the D-amino acid oxidase is further purified). The final reaction volume in the oxygen electrode cell is 2 ml. Final amounts of protein in the cell vary up to 5 mg depending on the level of activity being measured. Rates of oxygen consumption (after substraction of any drift in the bases line) are measured.

Example results obtained under the conditions described above are as follows. Wild-type R. gracilis D-amino acid oxidase exhibits no detectable ability to oxidise D-phosphinothricin and only low activity (∼30 nmol/min/mg) when D-aspartate is used as substrate (as compared to control rates of > 40 umol/min/mg observed when using 25 mM D-alanine as substrate). The F58K mutant form exhibits some low activity versus phosphinthricin (>∼15 nmol/min/mg) and moderate activity (∼1.8 umol/min/mg) with D-asparatate. The F58KM213S double mutant form exhibits a very high activity versus D-aspartate of ∼ 40 umol/min/mg and, versus, DL phosphinothricin, a high level of activity of ∼ 3.2 umol/min/ mg. The Km for D-phosphinothricin of the F58KM213S double mutant is estimated to be ∼ 12 mM. The triple mutant F58K, M213S, Y223H exhibits a moderate activity of ∼ 0.4 umol/ min/mg versus D-phosphinothricin and - 0.7 umol/min/mg versus D-aspartate.

In control experiments the pure L-form is not oxidised at detectable levels and, depending on concentration, the pure D form is oxidised at up to twice the rate that the DL racemate is.

Additional results are also obtained using a higher throughput assay method based on the method of Konno in 'Methods for the Detection of D-Amino-Acid Oxidase'. Biol. Proced. Online.(1998) May 14; 1: 27-31. This is an especially useful method for the initial selection of mutants prior to more accurate analysis using the oxygen electrode assay. Random/Site Directed mutants of the D-amino acid oxidase gene obtained as described above are cloned into PET24 or PET21 vector (Novagen) as NdeI/EcoRI fragments and heat-shock transformed into BL21-CodonPlus^{®}(DE3)-RP Competent Cells (Stratagene). Following selection and plating out, individual colonies are picked and grown in 1ml of L Broth (+Kanamycin or Ampilicillin+Chloramphenicol) at 30°C overnight in a 96 well plate containing large 2 ml wells. The plate is then subjected to low-speed centrifugation, the cells are spun to the bottom of the plate and the supernatant carefully removed. The cells are resuspended by vortexing in 0.5ml of fresh L broth (no antibiotic) and left to incubate and shake for a further 2 hours at 30°C. A further 0.5ml of L Broth + 4ul IPTG is added and the plate put back in shaking incubator (30°C) for 3 hours. The plate is once more centrifuged so that the cells are spun down, the supernatant is removed and the plate frozen at -80°C for 10 min. The plate is then restored to lab temperature and the cell pellets lysed prior to enzyme assay. 0.4 ml of CelLyticB (Sigma) containing 1mg/ml of lyzozyme is added to each well and left for 10 min. A glass bead is added to each well and then the wells sealed as a block to be ground in bead mill for 2 minutes. The plate is then centrifuged to separate out the course debris. 10 ul of the resultant supernatant extract is then assayed by adding to 30 ul of test D-amino acid (e.g. ammonium or potassium salts of D-glutamate, D-aspartate or DL glufosinate at, for example, 5, 10, 25, 50/100 or 200mM in water) along with 30 ul of 0.133M Pyrophosphate buffer at pH ∼ 8.3 (including 1ul/ml of Beta-mercaptoethanol and 5mg/ml Catalase), 20ul 0.1 mM FAD and 10ul of 70% methanol. The plate is then incubated to allow the assay to run at room temperature for 10-60 min and then the reaction in each well stopped with the addition of 100ul of 10% TCA. 50 ul is then removed from each well into the corresponding well of a new plate where each well contains 50ul 5M KOH. 50 ul 0.5M HCl containing 0.5% 'Purpald' (4-amino-5-hydrazino-1,2,4-triazole-3-thiol) is then added to each well and the plate is left for 15minutes at room temperature. After this time 50 ul of 0.2M KOH containing 0.75% potassium periodate is added to each well and finally 5 ul of isopropanol is added to each well to prevent from forming. The optical density of each well of the plate late is then measured at 550nm. High levels of D-amino acid oxidase activity correspond with high OD readings. The assay can be quantified using standard additions of keto acids and specific activities are calculated on the basis of protein concentrations measured using standard methods such as the Bradford or Lowry methods. Specific activities of DAMOX are estimated on the basis of the percentage of the total protein in extracts which is D-amino acid oxidase (as estimated by Coomassie stained SDS PAGE). Using the above assay it is shown, for example, at a substrate concentration of 25 mM D-phosphinothricin, that the F58K, M213T double mutant form of *R. gracilis* D-amino acid oxidase is about 2-5 times more active than is the F58K, M213S form (see Table 1)

**Table 1**

| | | |
|---|---|---|
| **Example results obtained from a plate-based assay comparing the activities of mutant forms of D-amino acid oxidase with D-phosphinothricin as substrate.** The assay is run under the conditions described above using 25 mM DL phosphinothricin as substrate. Extracts containing the wild-type form of the enzyme do not produce any detectable colour under these conditions. The optical density at 550 nm obtained after a 30 min assay of the extract of the F58K, M213T mutant form of *R. gracilis* DAMOX is more than double that obtained from a similar extract of the F58K, M213S mutant form. Since the assay becomes non-linear at high optical densities, it is estimated that the F58K, M213T mutant form is anything from 2-5X more active than the F58K, M213S form under the assay conditions. | | |

| **Mutant** | **Experiment** | **Absorbance at 550 nm** |
|---|---|---|
| F58K, M213T | 1 | 2.2019 |
| F58K, M213T | 2 | 2.2966 |
| F58K, M213T | 3 | 1.5633 |
| F58K, M213T | 4 | 1.4484 |
| F58K, M213T | Average | **1.8775** |
| | | |
| F58K, M213S | 1 | 0.9843 |
| F58K, M213S | 2 | 0.8374 |
| F58K, M213S | Average | **0.9109** |
| | | |
| F58H, M213S | 1 | 0.5982 |
| F58H, M213S | 2 | 0.6030 |
| F58H, M213S | Average | **0.6006** |

### Example 11. Random mutagenesis and selection to generate further mutants of the F58K D-amino acid oxidases genes encoding enzymes with improved specificity (kcat/ Km) for the oxidation of D-phosphinothricin

A DNA sequence, codon-optimized for expression in yeast and encoding the F58K mutant form or F58K,M213S or F58K,M213T double-mutant form of the *Rhodotorula gracilis* D-amino acid oxidase is cloned into Invitrogen's pYES6/CT shuttle vector as a HindIII/PmeI fragment downstream of the GAL1 promoter. Similarly, these DNA sequences are cloned into the pAUR123 protein expression shuttle vector (Panvera) as an XbaI fragment downstream of the ADHI constitutive promoter. Construction of these vectors is performed in E.coli followed by transformation into S288C Saccharomyces cerevisiae. Where appropriate, the PAT gene is used to replace the blasticidin or aureobasidin antibiotic resistance genes on the pYES6/CT/pAUR123 vectors respectively and DL phosphinothricin rather than antibiotic used to maintain selection. Further mutant variants of D-amino acid oxidase are created using various methods of mutagenesis. For example, multiple variants of the D-amino acid oxidase coding sequence are generated by Mn2+-poisoned PCR, the mixed population is cloned in front of the GAL1 or ADH1 promoters of the two shuttle vectors, transformed into yeast, the yeast is further transformed with a PAT gene and selection made based upon the ability of the new sequence to confer upon yeast the ability to grow more rapidly in a minimal medium on phosphinothricin as as major nitrogen source. Alternatively mutation and selection is carried out directly on the transformed yeast. For example, yeast transformed with the above plasmids are grown up in a fermenter in the presence of a chemical mutagen such as EMS in a nitrogen-limited culture medium which contains 20-100 mM DL phosphinothricin and induced for D-amino acid oxidase expression (e.g. grown on galactose as carbon source). After successive subculturings, subcultures growing fastest on phosphinothricin as major N source are identified, plated out and the D-amino acid oxidase coding sequences subcloned, sequenced and expressed in E.coli for further characterisation.

In a further, preferred, method mutagenesis is carried out on the two shuttle vectors by using amplification and passage through E.coli strain XL1-red. This strain is deficient in three primary DNA repair pathways, mut S, mut D and mut T. This results in - a 5000 fold increase in mutation rates during DNA replication. The protocol used is according to Stratagene. For example, 10 ng of shuttle vector is transformed into E.coli strain XL1-red, cells are grown up and then plated out onto L-Broth agar containing ampicillin for 24h. From each plate > 200 transformant lots of colonies are pooled by scraping the colonies off the plate into L broth and then 1 in 100 and 1 in 1000 dilutions are grown and successively subcultured in L-broth/ampicillin at 37°C for 1-2 weeks so that a large number of cell- divisions have ensued. A similar procedure is carried out starting from a number of plates. Minipreps of shuttle vector DNA are prepared from cells grown overnight and transformed back into yeast. The transformed yeast are grown up and colonies containing improved D-amino acid oxidases selected as described above.

Alternatively, D-amino acid oxidase expression and selection is carried out in some microorganism other than yeast and, for example, under expression control of the t7 promoter of a pET vector in an E.coli lysogen. In this case, the D-amino acid oxidase coding sequence (optionally mutagenised by Mn2+-poisoned PCR) is cloned into a pET vector, transformed into E.coli XL1 red and after passage for a number of generations, transformed back into an E.coli lysogen such as E.coli BL212 DE3. Individual colonies may then be picked, replica plated, grown, induced with IPTG, lysed and screened for the desired substrate activity versus D-phosphinothricin using methods known in the art (for example, a fluorimetric screen for peroxide generation or a colorimetric assay for 2 keto acid or ammonia generation). Alternatively, the transgenic E.coli lines are further transformed so that they express the PAT gene (for example, constitutively) and induced with IPTG so that they express the 'test' mutant D-amino acid oxidase. Induced lines which exhibit the best growth on minimal medium provided with phosphinothricin (or, optionally, a phosphinothricin analogue) as the major N source (optionally a small amount of ammonium ions are included) are selected.

Optionally the media used for selection of yeast contain a low concentration of solvent (e.g 0.1% DMSO).

### Example 12. Production of D-phosphinothricin in an enantiomerically pure form

E.coli BL21 DE3 codon plus RIL is transformed with Novagen pET 24A having the PAT coding sequence (A02774) cloned for (untagged) expression behind the T7 promoter. These cells are grown to a density of ∼ 40 OD₆₀₀ₙₘ in a 10 litre fermentor of LCM50 medium containing kanamycin , induced with 0.2mM IPTG, harvested by low speed centrifugation and quickly transferred into minimal media containing 9.91g of the ammonium salt of D/L phosphinothricin (PPT).

### Minimal media (in 1 litre) is.

Na₂HPO₄ (6g), KH₂PO₄ (3g), NaCl (1g), NH₄Cl (1g) were dissolved in water and autoclaved and the following solutions were added after filter sterilisation:
CaCl₂ (1ml of 14.7g/l), MgSO₄ (1ml of 246.5g/l), Thiamine.HCl (5ml of 1mg/ml)
Glucose (30ml of 20% solution autoclaved separately), DMSO 0.5ml.

Fermentation details are as follows. A 10 litre fermenter of LCM 50 medium is inoculated with an LB broth-grown inoculum (200ml) of E.coli BL21 DE3 codon plus RIL containing the PAT gene and is maintained at 30°C, 200 rpm stirring rate, pH6.5, oxygen concentration 50% air-saturated. After ∼ 12 h the culture grows to an OD 600nm of ∼ 30. The culture is then induced for PAT expression by the addition of 0.2mM IPTG. After 1.5 h, the culture typically grows further to an OD 600nm of ∼ 40, before the cells are harvested by centrifugation and washed in 8 litres of minimal medium. The cells are spun once again and resuspended to a final volume of 10 litres in the fermenter in minimal media containing 9.91 g of the commercially available ammonium salt of D/L - phosphinothricin and a further 0.2mM IPTG. The temperature is increased to 37°C and samples of the fermenter medium monitored by proton and phosphorous NMR in order to determine a) when the glucose levels have dropped substantially and need replenishing and b) the extent of conversion of phosphinothricin to n-acetyl phosphinothricin. Over the course of ∼ 12 h, ∼ 500g of glucose are added to the fermenter. The formation of n-acetyl phosphinothricin is observed to start after a few hours and by ∼ 20 h reaches > 93 % conversion of the L-PPT (46.5 %v of the D/L) to N-acetyl-L-PP. The fermentation medium is harvested soon thereafter with the cells being remove by low-speed centrifugation.

D-PPT is purified from the fermentation medium using ion-exchange chromatography. The fermentation medium (∼ 9.5 1) is stored at 4°C. It is mixed with 900ml of Dowex 50W-X8 200-400 mesh cation exchange resin (pre-prepared with HCl) in the H⁺ form such that the pH of the supernatant above the resin drops to ∼ pH 3.0. The Dowex resin is allowed to settle out under gravity and the supernatant together with a 21 water rinse of the Dowex resin is decanted off and then centrifuged to clarify. The washed Dowex is discarded (to eventually be recypled). The clarified supernatant is then extracted via a separating funnel with ethyl acetate (1/4 of the volume of supernatant) and the aqueous fraction (∼ 121) retained. A further 2.31 of H⁺ form Dowex 50W-X8 resin is then added and stirred with the - 121. The resin is then allowed to settle out. The pH of the supernatant above the resin is ∼ pH 1.6 at this stage. The supernatant is decanted off and discarded and the resin washed with ∼ 12 litres of water and, again, allowed to settle out. Again, the supernatant is discarded and the resin is poured onto a sintered Buchner funnel filter and rinsed with a futher ~ 4.51 of water (to remove most of the residual N-acetyl-phosphinothricin). The major D-phosphinothricin- containing fraction is eluted from the resin with 151 of 0.4M ammonium hydroxide, followed by a 1.41 water rinse of the resin. The pH of this D-phosphonothricin-containing fraction is ∼ 11.4. Optionally, this is reduced to - pH 10 by the addition of , for example, - 0.13 Moles of acetic acid and ~ 600ml of cation exchanger resin in the H⁺ form. If added, the resin is allowed to settle out. The D-phosphinothricin (supernatant) fraction is then loaded on to a 565ml (5 x 28cm) column of Dowex 1X8 - 400 mesh anion exchange resin in the OH- form (preequilibrated with NaOH and washed with water). A OM - 0.32M ammonium acetate gradient is applied to the column over 17 column bed volumes. 55ml fractions are collected throughout. The fractions are monitored by UV at 215nm and also by proton and 31P NMR. This analysis indicates that highly pure phosphinothricin is eluted between fractions 39 and 78. N-acetyl phosphinothricin is eluted as unbound material and early in the gradient and some glutamate elutes later in fractions 79 - 90.
Fractions 63 to 78 (corresponding to 6-7.6 bed volumes) constitute the bulk of highly pure phosphinothricin. The phosphinothricin fractions are freeze dried and found to be pure by proton and phosphorous NMR (no other peaks visible apart from acetate, > 95% of the organic material is phosphinothricin), although, based upon discrepancies between calculated and observed dry weights it is found that, typically, some residue of inorganic salts (for example ammonium chloride) remain in the phosphinothricin samples. For practical purposes, when the D-phosphinothricin is used (for example to spray on plants) the inorganics can be taken to be inert and only need to be taken account to adjust calculated concentrations when D-phosphinothricin solutions are made of from weighed dry samples.

It is expected that the phosphinothricin isolated according to the above method should be substantially enantiomerically pure D-phosphinothricin. This is verified according to the fluorescent HPLC analysis method of Hori et al. (2002) J.Chrom. B 776, 191-198. For example, 50 ul of either commercial DL phosphinothricin (0.01-10 ug/ ml) or of sample is dissolved in 0.1 M Borate buffer pH8.5 and mixed with 200ul of the same Borate buffer. 50ul of 18mM FLEC ((+)-1-(9-fluorenyl) ethyl chloroformate) is then added and the mixtures further incubated for 30 mins at 40°C. Excess FLEC is removed by shaking for 3mins with 500ul of ethyl acetate. 100ul of the bottom aqueous layer is removed for HPLC analysis.

An Inertsil ODS2 (15 x 4.6) 5uM partical HPLC column is equilibrated with 77% 10mM aqueous ammonium acetate (pH5.0): 23% Acetonitrile at a flow rate of 0.8ml/min. A 2ul sample is injected onto the column and run isocratically over 60mins and is monitored using fluorescence detection with excitation at 260nm and emission wavelength at 305nm. It is observed that the D & L isomers of phosphinothricin are clearly separated and elute at 12.4 and 13.4 mins respectively. A sample of the D phosphinothricin isolated according to the current method is run and is estimated to be at a better than 99% enantiomeric excess. This is estimated on the basis of spiking with known quantities of commercial DL phosphinothricin and observing how small an increase in the right-hand, 13.4 minute peak is detectable against the background of the apparently single, 12.4 min peak yielded by the sample.

In addition, the HPLC method is used to estimate the amount of phosphinothricin on the basis of peak integration and comparison with a standard curve. Additionally total amounts of phosphinothricin are estimated by integration of NMR signals.

It is estimated that, in total, from the starting - 9.91g of DL racemate, ∼ 1.9 g (38% yield) of pure D-phosphinothricin ammonium salt in an enantiomeric excess of > 99% is produced. 50-70% of the dry weight of the sample comprises inorganic salts which are carried through. Optionally these are removed by further steps of ion exchange and freeze drying (following exchange to volatile salts).

## Claims

1. A method of producing male or female sterile plants comprising the steps of transforming plant material with a polynucleotide which encodes at least one enzyme which reacts with a non-phytotoxic substance to produce a phytotoxic one, and regenerating the thus transformed material into a plant, wherein the said non-phytotoxic substance is applied to the plant up to the time of male or female gamete formation and/or maturation, so that the non-phytotoxic substance provides for the production of a phytotoxic one which selectively prevents the formation of or otherwise renders the said gametes non-functional, wherein the enzyme is expressed preferentially in either male or female reproductive structures and the non-phytotoxic substance is a D-alpha amino acid, **characterised in that** the enzyme is a D-amino acid oxidase obtained from Rhodotorula gracilis and encoded by the nucleic acid sequence as depicted in SEQ ID NO.3, which enzyme is a mutated version of the protein encoded by SEQ ID NO. 3 and comprises a lysine at position 58 and at position 213 an amino acid selected from the group consisting of Thr, Gln, and Gly; and/or at position 238 an amino acid selected from the group consisting of Thr, Gln and Gly and/or at position 223 an amino acid selected from the group consisting of Thr, Cys, Gly, Gln and/or Asn.

2. A method according to claim 1, wherein the said non-phytotoxic substance is applied in mixture along with at least one further substance which is selected from the group consisting of safeners, gametocides, glutathione-S-transferase inducers, cytochrome P450 inducers, herbicides, fertilizers, nematocides, synergists, insecticides, fungicides, hormones, plant-growth regulators and cytochrome P450 inhibitors.

3. A method according to claim 1 or 2, wherein the non-phytotoxic substance is applied foliarly and is a phloem mobile and metabolically stable oxidiseable substrate of the enzyme, wherein the enzyme provides the phytotoxic product, as a direct or indirect one from the non-phytotoxic substance.

4. A method according to the preceding claim, wherein the phytotoxic product is an indirect one produced in the form of peroxide and/or a super oxide anion.

5. A method according to either of claims 3 or 4, wherein the non-phytotoxic substance is D-aspartate or D-glutamate and the enzyme oxidises the said amino acid to a 2-keto acid with concomitant reduction of oxygen to a peroxide anion.

6. A method according to the previous claim where the amino acid at position 213 is Thr.

7. A method according to anyone of claims 3-6, wherein the enzyme is targeted to other than the peroxisome.

8. A method according to claim 1 or 2, wherein the non-phytotoxic substance is either the D enantiomer of phosphinothricin or a D enantiomer of bialaphos.

9. A method according to either of claims 1 or 2, wherein the non-phytotoxic substance is comprised within a mixture, which contains a phytotoxic substance and wherein the enzyme oxidises an amino acid to a 2-keto acid with concomitant reduction of oxygen to a peroxide anion.

10. A method according to any one of claims 8 or 9, wherein the mixture comprises both D and L phosphinothricin and the plant material expresses a PAT gene substantially only in green tissues and/or in floral tissue which produce gametes being other than those that are rendered non-functional.

## Patentansprüche

1. Verfahren zum Herstellen männlicher oder weiblicher steriler Pflanzen, umfassend die Schritte des Transformierens von Pflanzenmaterial mit einem Polynukleotid, das für mindestens ein Enzym kodiert, das mit einem nicht-phytotoxischen Stoff reagiert, um einen phytotoxischen herzustellen, sowie des Regenerierens des auf diese Weise hergestellten Materials in eine Pflanze, wobei der nicht-phytotoxische Stoff an der Pflanze bis zum Zeitpunkt der männlichen oder weiblichen Gametenbildung und/oder Reifung angewandt wird, so dass der nicht-phytotoxische Stoff die Herstellung eines phytotoxischen besorgt, der selektiv die Bildung der Gameten verhindert oder diese auf sonstige Weise funktionslos macht, wobei das Enzym vorzugsweise entweder in männlichen oder weiblichen reproduktiven Strukturen exprimiert wird und der nicht-phytotoxische Stoff eine D-alpha-Aminosäure ist, dadurch charakterisiert, dass das Enzym eine D-alpha-Aminosäureoxidase ist, die von Rhodotorula gracilis gewonnen wurde und von der Nukleinsäure, wie in SEQ ID Nr.: 3 gezeigt, kodiert wird, wobei das Enzym eine mutierte Version des Proteins ist, das von SEQ ID Nr.: 3 kodiert wird und ein Lysin an der Position 58, an der Position 213 eine Aminosäure ausgewählt aus der Gruppe bestehend aus Thr, Gln und Gly, und/oder an der Position 238 eine Aminosäure ausgewählt aus der Gruppe bestehend aus Thr, Gln und Gly und/oder an der Position 223 eine Aminosäure ausgewählt aus der Gruppe bestehend aus Thr, Cys, Gly, Gln und/oder Asn umfasst.

2. Verfahren gemäß Anspruch 1, wobei der nicht-phytotoxische Stoff in einer Mischung mit mindestens einem weiteren Stoff angewandt wird, der aus der Gruppe bestehend aus Safenern, Gametociden, Glutathion-S-Transferaseinduktoren, Cytochrom P450-Induktoren, Herbiciden, Düngern, Nematociden, Synergisten, Insektiziden, Fungiziden, Hormonen, Pflanzenwachstumsregulatoren und Cytochrom P450-Inhibitoren ausgewählt wird.

3. Verfahren gemäß Anspruch 1 oder 2, wobei der nicht-phytotoxische Stoff an Blättern angewandt wird und ein Phloemmobiles und metabolisch stabiles oxidierbares Substrat des Enzyms ist, wobei das Enzym ein phytotoxisches Erzeugnis besorgt, als direktes oder indirektes ausgehend vom nicht-phytotoxischen Substrat.

4. Verfahren gemäß dem vorhergehenden Anspruch, wobei das phytotoxische Erzeugnis ein indirektes ist, das in der Form eines Peroxids und/oder eines Superoxidanions hergestellt wird.

5. Verfahren gemäß einem der Ansprüche 3 oder 4, wobei der nicht-phytotoxische Stoff D-Aspartat oder D-Glutamat ist und das Enzym diese Aminosäure zu einer 2-Ketosäure oxidiert unter gleichzeitiger Reduktion von Sauerstoff zu einem Peroxidanion.

6. Verfahren gemäß dem vorhergehenden Anspruch, wobei die Aminosäure in der Position 213 Thr ist.

7. Verfahren gemäß einem beliebigen der Ansprüche 3 bis 6, wobei das Enzym wo anders als auf das Peroxisom hin zielgesteuert wird.

8. Verfahren gemäß Anspruch 1 oder 2, wobei der nicht-phytotoxische Stoff entweder das D-Enantiomer von Phosphinothricin oder ein D-Enantiomer von Bialaphos ist.

9. Verfahren gemäß einem der Ansprüche 1 oder 2, wobei der nicht-phytotoxische Stoff von einer Mischung umfasst ist, die einen phytotoxischen Stoff enthält und wobei das Enzym eine Aminosäure zu einer 2-Ketosäure unter gleichzeitiger Reduktion von Sauerstoff zu einem Peroxidanion oxidiert.

10. Verfahren gemäß einem beliebigen der Ansprüche 9 oder 10, wobei die Mischung sowohl D- als auch L-Phosphinothricin umfasst und das Pflanzenmaterial ein PAT-Gen im wesentlichen nur in grünen Geweben und/oder in Blumengewebe umfasst, welches Gameten produziert, die anders sind, als diejenigen die funktionslos gemacht wurden.

## Revendications

1. Procédé de production de plantes stériles mâles et femelles comprenant les étapes de transformation de matériel végétal avec un polynucléotide qui code au moins une enzyme qui réagit avec une substance non phytotoxique pour produire une substance phytotoxique, et de régénération du matériel ainsi transformé en une plante, où ladite substance non phytotoxique est appliquée à la plante jusqu'au moment de la formation et/ou la maturation de gamètes mâles ou femelles, de sorte que la substance non phytotoxique permet la production d'une substance phytotoxique qui empêche sélectivement la formation de ou rend d'une autre manière lesdits gamètes non fonctionnels, où l'enzyme est exprimée préférentiellement dans les structures reproductrices mâles ou femelles et la substance non phytotoxique est un D-alpha aminoacide, **caractérisé en ce que** l'enzyme est une D-aminoacide oxydase obtenue à partir de Rhodotorula gracilis et codée par la séquence d'acide nucléique représentée dans SEQ ID NO. 3, laquelle enzyme est une version mutée de la protéine codée par SEQ ID NO. 3 et comprend une lysine à la position 58 et à la position 213 un aminoacide choisi dans le groupe consistant en Thr, Gln et Gly, et/ou à la position 238 un aminoacide choisi dans le groupe consistant en Thr, Gln et Gly et/ou à la position 223 un aminoacide choisi dans le groupe consistant en Thr, Cys, Gly, Gln et/ou Asn.

2. Procédé selon la revendication 1 où ladite substance non phytotoxique est appliquée en mélange avec au moins une autre substance qui est choisie dans le groupe consistant en les agents d'innocuité, les gamétocides, les inducteurs de glutathion-S-transférase, les inducteurs de cytochrome P450, les herbicides, les engrais, les nématocides, les agents de synergie, les insecticides, les fongicides, les hormones, les régulateurs de croissance des plantes et les inhibiteurs de cytochrome P450.

3. Procédé selon la revendication 1 ou 2 où la substance non phytotoxique est appliquée par application foliaire et est un substrat oxydable de l'enzyme mobile dans le phloème et métaboliquement stable, où l'enzyme forme le produit phytotoxique, sous forme de produit phytotoxique direct ou indirect à partir de la substance non phytotoxique.

4. Procédé selon la revendication précédente où le produit phytotoxique est un produit phytotoxique indirect produit sous forme d'anion peroxyde et/ou superoxyde.

5. Procédé selon l'une ou l'autre des revendications 3 et 4 où la substance non phytotoxique est le D-aspartate ou le D-glutamate et l'enzyme oxyde ledit aminoacide en un 2-cétoacide avec réduction concomitante de l'oxygène en un anion peroxyde.

6. Procédé selon la revendication précédente où l'aminoacide à la position 213 est Thr.

7. Procédé selon l'une quelconque des revendications 3-6 où l'enzyme est dirigée vers une autre cible que le peroxysome.

8. Procédé selon la revendication 1 ou 2 où la substance non phytotoxique est l'énantiomère D de la phosphinothricine ou un énantiomère D du bialaphos.

9. Procédé selon la revendication 1 ou 2 où la substance non phytotoxique est comprise dans un mélange, qui contient une substance phytotoxique et où l'enzyme oxyde un aminoacide en un 2-cétoacide avec réduction concomitante de l'oxygène en un anion peroxyde.

10. Procédé selon l'une quelconque des revendications 8 et 9 où le mélange comprend de la D phosphinothricine et de la L phosphinothricine et le matériel végétal exprime un gène PAT sensiblement seulement dans les tissus verts et/ou dans le tissu floral qui produisent des gamètes différents de ceux qui sont rendus non fonctionnels.
